# EUROPEAN PATENT SPECIFICATION

(11) **EP 1 694 697 B1**
(45) Date of publication and mention of the grant of the patent: **29.12.2010**
(21) Application number: 04797464.7
(22) Date of filing: 19.11.2004
(51) Int. Cl.: C07K 14/205, C12N 15/11, G01N 33/569, C07K 16/12, A61K 39/02

(54) **SURFACE-LOCATED CAMPYLOBACTER JEJUNI POLYPEPTIDES**
AN DER OBERFLÄCHE BEFINDLICHE CAMPYLOBACTER JEJUNI-POLYPEPTIDE
POLYPEPTIDES DE CAMPYLOBACTER JEJUNI LOCALISES EN SURFACE

(30) Priority: 21.11.2003 DK 200301726; 25.11.2003 US 524617 P
(43) Date of publication of application: 30.08.2006
(73) Proprietor: Ace Biosciences A/S, DK-5220 Odense SØ (DK)
(72) Inventor: SCHROTZ-KING, Petra, 5260 Odense SØ (DK); SKAARUP, Crawford, Janne, 5772 Kværndrup (DK); NYBORG, Nielsen, Pia, 5230 Odense M (DK); PROKHOROVA, Tatyana, A., 5592 Ejby (DK)
(74) Representative: Høiberg A/S
(86) International application number: PCT/DK2004/000803
(87) International publication number: WO 2005/049641

(56) References cited:
- CA-A1- 2 296 869
- US-A- 5 470 958
- DATABASE GSP 16 June 2003 (2003-06-16), XP002334809 retrieved from EBI Database accession no. ABU26391 -& WO 02/077183 A (ELITRA PHARMACEUTICALS, INC; WANG, LIANGSU; ZAMUDIO, CARLOS; MALONE, C) 3 October 2002 (2002-10-03) cited in the application
- DATABASE UniProt 15 October 2000 (2000-10-15), XP002326090 retrieved from EBI Database accession no. Q9PJ34 -& PARKHILL J ET AL: "THE GENOME SEQUENCE OF THE FOOD-BORNE PATHOGEN CAMPYLOBACTER JEJUNIREVEALS HYPERVARIABLE SEQUENCES" NATURE, MACMILLAN JOURNALS LTD. LONDON, GB, vol. 403, 10 February 2000 (2000-02-10), pages 665-668, XP000906767 ISSN: 0028-0836 cited in the application
- DATABASE UniProt 1 October 2000 (2000-10-01), XP002334638 Database accession no. Q9PM33 & PARKHILL J ET AL: "THE GENOME SEQUENCE OF THE FOOD-BORNE PATHOGEN CAMPYLOBACTER JEJUNIREVEALS HYPERVARIABLE SEQUENCES" NATURE, MACMILLAN JOURNALS LTD. LONDON, GB, vol. 403, 10 February 2000 (2000-02-10), pages 665-668, XP000906767 ISSN: 0028-0836 cited in the application
- DATABASE UniProt 1 October 2000 (2000-10-01), XP002334639 retrieved from EBI Database accession no. Q9PNS2 & PARKHILL J ET AL: "THE GENOME SEQUENCE OF THE FOOD-BORNE PATHOGEN CAMPYLOBACTER JEJUNIREVEALS HYPERVARIABLE SEQUENCES" NATURE, MACMILLAN JOURNALS LTD. LONDON, GB, vol. 403, 10 February 2000 (2000-02-10), pages 665-668, XP000906767 ISSN: 0028-0836 cited in the application
- DATABASE UniProt 1 October 2000 (2000-10-01), XP002334640 retrieved from EBI Database accession no. Q9PI85 & PARKHILL J ET AL: "THE GENOME SEQUENCE OF THE FOOD-BORNE PATHOGEN CAMPYLOBACTER JEJUNIREVEALS HYPERVARIABLE SEQUENCES" NATURE, MACMILLAN JOURNALS LTD. LONDON, GB, vol. 403, 10 February 2000 (2000-02-10), pages 665-668, XP000906767 ISSN: 0028-0836 cited in the application
- ALLAN E ET AL: "Genes to genetic immunization: identification of bacterial vaccine candidates" METHODS : A COMPANION TO METHODS IN ENZYMOLOGY, ACADEMIC PRESS INC., NEW YORK, NY, US, vol. 31, no. 3, November 2003 (2003-11), pages 193-198, XP004457831 ISSN: 1046-2023

## Description

### Field of the invention

The present invention relates to cell-surface-located polypeptides of Campylobacter jejuni and their use in immunisation against Campylobacter infection, in diagnosis of Campylobacter and in identification of compounds with anti-Campylobacter activity.

### Background of the invention

### Occurrence of Campylobacter infections

Campylobacter, a Gram-negative microaerophilic bacterium, was first identified as a human pathogen in 1973. It has since become the most common bacterial cause of diarrhoeal illness in the developed world, causing more disease than the more traditionally recognised food-borne pathogens, Shigella species (spp). and Salmonella spp. combined. Of the different disease-causing Campylobacter strains, C. jejuni is the most important, being responsible for 99% of cases of campylobacteriosis. At a global level, surveillance has indicated a steady rise in the number of reported cases of campylobacteriosis since this organism was first recognised as a pathogen. Indeed, the World Health Organisation now recognises bacteria causing campylobacteriosis to be the most important agents of enteritis in the world. International public health officials estimate that C. jejuni alone causes 400 to 500 million cases of diarrhoea world-wide each year, and it is the number one food-borne pathogen in the U.S. Recent data for the year 2000 illustrate the significance of Campylobacter with respect to other more publicised causes of food borne illness. Campylobacter accounts for more cases, hospitalisations and deaths than Salmonella or E. colimediated food-borne illnesses. Amongst the data set, Campylobacter accounts for greater than 55% of cases and 33% of hospitalisations.

### Symptoms of Campylobacter infections

Diarrhoea is the most consistent and prominent manifestation of campylobacteriosis. It is often bloody. Typical symptoms of C. jejuni infection also include fever, nausea, vomiting, abdominal pain, headache, and muscle pain. A majority of cases are mild and do not require hospitalisation and may be self-limited. However, C. jejuni infection can be severe and life-threatening. Death is more common when other diseases (e.g. cancer, liver disease, and immunodeficiency-related diseases) are present. Children under the age of five and young adults aged 15-29 are the age groups most frequently affected. The incubation period (the time between exposure on onset of the first symptom) is typically two to five days, but onset may occur in as few as 2 days or as long as 10 days after ingestion. The illness usually lasts no more than one week; however, severe cases may persist for up to three weeks (CDC Guidelines for confirmation of food-borne disease outbreaks. MMWR, 1996; 45:59).

### Long-term consequences of Campylobacter

Campylobacter infection can sometimes have long-term consequences. Some patients may develop a disease, called Guillain-Barré syndrome, that affects the nerves of the body following campylobacteriosis. Although rare, it is the most common cause of acute generalised paralysis in the Western world. It begins several weeks after the diarrhoeal illness in a small minority of Campylobacter patients. It occurs when a person's immune system generates antibodies against components of Campylobacter and these antibodies attack components of the body's nerve cells because they are chemically similar to bacterial components.¹ Guillain-Barré syndrome begins in the feet and spreads up the body. Prickling sensations give way to weakness that may lead to paralysis. It lasts for weeks to months and often requires intensive care. Full recovery is common, however patients may be left with severe neurological damage. Approximately 15% of Guillain-Barré patients remain bedridden or wheelchair-bound after one year. It is estimated that approximately one in every 1000 (0.1 %) reported campylobacteriosis cases leads to Guillain-Barré syndrome. As many as 40% of Guillain-Barré syndrome cases in the UK occur following campylobacteriosis.²
Miller Fisher Syndrome is another, related neurological syndrome that can follow campylobacteriosis and is also caused by immunologic mimicry.' In Miller Fisher syndrome, the nerves of the head are affected more than the nerves of the body.
Another chronic condition that may be associated with Campylobacter infection is an arthritis called Reiter's syndrome. This is a reactive arthritis that most commonly affects large weight-bearing joints such as the knees and the lower back. It is a complication that is strongly associated with a particular genetic make-up; persons who have the human lymphocyte antigen B27 (HLA-B27) are most susceptible.
In addition, Campylobacter may also cause appendicitis or infect the abdominal cavity (peritonitis), the heart (carditis), the central nervous system (meningitis), the gallbladder (cholecystitis) the urinary tract, and the blood stream. References:
1. Ang CW et al. (2001) Infect Immun.69(4):2462-2469.
2. Rees et al. (1995) N Engl J Med 333:1374-1379.

### Treatment of campylobacteriosis

Patients with campylobacteriosis should drink plenty of fluids as long as the diarrhoea lasts in order to maintain hydration. Antidiarrhoeal medications such as loperamide may allay some symptoms. Campylobacter is usually a self-limited illness, but when it is identified, specific treatment with antibiotics is indicated, as treatment may shorten the course of the illness. In more severe cases of gastroenteritis, antibiotics are usually begun before culture results are known. Macrolide antibiotics (erythromycin, clarithromycin, or azithromycin) are the most effective agents for C. jejuni. Fluoroquinolone antibiotics (ciprofloxacin, levofloxacin, gatifloxacin, or moxifloxacin) can also be used.
However, resistance of Campylobacter to antimicrobial agents has been reported in many countries and is on the rise (Pedungton and Kaneene (2003) J. Vet. Med. Sci. 65(2):161-170). Quinolone-resistance is on the rise in Europe, Asia and the US. The increase of resistance is at least partially related to the use of antibiotics in poultry feed (Smith et al. (1999) N Engl J Med 340:1525-1532.

### Novel strategies for the treatment, prevention and diagnosis of Campylobacter

Because of the increase in incidence and the widespread occurrence of resistance, there is a considerable need for the development of new effective products for the treatment and prevention of Campylobacter infections. On one hand, due to the occurrence of resistance, there is a need for novel anti-Campylobacter compounds. On the other hand, observational and experimental studies have provided evidence of acquired immunity developing in humans, lending support to the concept of vaccine development, in particular for risk groups (Scott and Tribble (2000) In: Campylobacter, 2nd ed. Ed. by Nachamkin and Blaser, American Society for Microbiology, pp. 303-319). In addition, there is a need for novel rapid and reliable methods for diagnosis of Campylobacter infections.
These objectives can be accomplished through the identification and use of suitable Campylobacter jejuni polypeptides that can function as targets, i.e. targets for the immune system and/or for antibodies, targets for cytotoxic inhibitors, or targets for indicator moieties in diagnosis.

WO 02/077183 (Elitra) relates to sequences of antisense nucleic acids which inhibit the proliferation of prokaryotes. The nucleic acids can, inter alia, be used to screen for homologous nucleic acids that are required for proliferation in cells other than Staphylococcus aureus, Salmonella typhimurium, Klebsiella pneumoniae, and Pseudomonas aeruginosa.

US 5,470,958 (Enteric Research Laboratories Inc.) relates to an antigenic composition including antigens obtainable from Campylobacter jejuni. The antigenic composition is disclosed for use as a vaccine to induce protective antibodies against both Campylobacter coli and Campylobacter jejuni.

CA 2,296,869 (Angela et al.) relates to proteins of Campylobacter jejuni involved in the adhesion and invasion of the said bacterium. It further relates to the assembly into a kit of the products disclosed, wherein said kit is disclosed for use in detection of the said bacterium by assaying for a J1pA protein or molecules, the presence of nucleic acid molecules or the presence of a lipoprotein or a lipid moiety on a polypeptide in a sample. Also, it relates to a composition for treatment of infectious diseases and to a vaccine.

### Summary of the invention

The present application in one embodiment relates to a composition comprising
- a polypeptide which comprises the sequence of SEQ ID NO:43 wherein X1 is V or A and X2 is A or T and X3 is T or A, or comprises an antigenic fragment selected from the group consisting of SEQ ID NO: 74-81 or a variant of said sequence having at least 75% sequence identity to said sequence,
- a polynucleotide comprising a sequence encoding said polypeptide,
- an expression vector comprising a sequence encoding said polypeptide,
- a recombinant virus or recombinant cell comprising said polynucleotide or said expression vector, or
- an antibody capable of binding said polypeptide,
for use as a medicament.

The present application in a further embodiment relates to an antibody capable of binding a polypeptide of SEQ ID NO:43 wherein X₁ is V or A and X₂ is A or T and X₃ is T or A for use as a medicament.

The present application in a further embodiment relates to a recombinant attenuated or reduced-virulence Escherichia coli or recombinant attenuated or reduced-virulence Salmonella cell transformed or transfected with a polynucleotide comprising a sequence encoding the polypeptide of SEQ ID NO:43 wherein X₁ is V or A and X₂ is A or T and X₃ is T or A, or encoding an antigenic fragment or variant of said sequence as defined in claim 1, for use as a medicament.

The present application in a further embodiment relates to use of
- a polypeptide which comprises the sequence of SEQ ID NO:43 wherein X₁ is V or A and X₂ is A or T and X₃ is T or A, or comprises an antigenic fragment or variant of said sequence as defined in claim 1,
- a polynucleotide comprising a sequence encoding said polypeptide,
- an expression vector comprising a sequence encoding said polypeptide, or
- a recombinant virus or recombinant cell comprising said polynucleotide or said expression vector,
for the preparation of a medicament for the immunisation of an animal or human being against Campylobacter, preferably Campylobacter jejuni, infections.

The present application in a further embodiment relates to use of an antibody capable of binding the polypeptide of SEQ ID NO:43 wherein X₁ is V or A and X₂ is A or T and X₃ is T or A, for the manufacture of a medicament for the treatment or prevention of Campylobacter, preferably Campylobacter jejuni, infections in an animal or human being.

The present application in a further embodiment relates to a method for detecting Campylobacter jejuni in a sample comprising the steps of
a. contacting said sample with an indicator moiety capable of specifically binding the polypeptide of SEQ ID NO:43 wherein X₁ is V or A and X₂ is A or T and X₃ is T or A, wherein the indicator moiety furthermore is capable of specifically binding intact Campylobacter jejuni cells, and
b. determining whether a signal has been generated by the indicator moiety, thereby detecting whether said sample contains Campylobacter jejuni.

The present application in a further embodiment relates to a method for identifying an inhibitor of the polypeptide of SEQ ID NO:43 wherein X₁ is V or A and X₂ is A or T and X₃ is T or A, comprising the steps of
a. providing two cells which differ in the level of the polypeptide of SEQ ID NO:43 wherein X₁ is V or A and X₂ is A or T and X₃ is T or A,
b. determining the sensitivity of said cells to a putative inhibitor, for instance by a growth assay as defined in claim 38, and
c. determining whether said two cells are differently affected by the presence of said putative inhibitor.

The present application in a further embodiment relates to a method for identifying a host-derived binding partner of the polypeptide of SEQ ID NO:43 wherein X₁ is V or A and X₂ is A or T and X₃ is T or A, or a fragment thereof as defined in claim 1, comprising the steps of
a. providing purified and electrophoretically separated host membranes blotted over to a membrane,
b. incubating said host membranes with the polypeptide of SEQ ID NO:43 wherein X₁ is V or A and X₂ is A or T and X₃ is T or A, or said fragment thereof,
c. detecting binding by using antibodies specific for the polypeptide of SEQ ID NO:43 wherein X₁ is V or A and X₂ is A or T and X₃ is T or A, or said fragment thereof, and
d. identifying the binding partner by elution from said blot membrane and subsequent analysis.

The present application relates to surface-located polypeptides of Campylobacter jejuni as defined in claim 1. In the context of this application, a 'surface-located' polypeptide is defined as a polypeptide which is at least partially (i.e. part of the polypeptide chain and/or part of the population of polypeptide molecules) localised outside the outer membrane of a Campylobacter jejuni cell. Thus, a surface-located polypeptide is a polypeptide which is fully or partially exposed to the space outside the outer-membrane. Surface-located polypeptides furthermore include all polypeptides or polypeptide fragments that can be identified in fractions obtained by low-pH surface-protein extraction as described herein.
Surface-located polypeptides are attractive targets for antibacterial therapy and/or diagnosis of bacterial infection, since the exposure of such polypeptides to the extracellular space means that compounds that interact with these polypeptides (e.g. compounds used to prevent, treat or diagnose bacterial infections) often do not need to enter or pass the outer membrane to be effective.
The determination of cell-surface localisation of a Campylobacter jejuni polypeptide can at present only be done experimentally and not by bioinformatics, as no common sorting signals or motifs are known for this localisation. It is possible to predict with some degree of certainty whether or not polypeptides enter the periplasm, but no general motif has been identified for surface-localisation of polypeptides, and therefore it is not possible to predict from the sequence alone whether any given periplasmic (or non-periplasmic) polypeptide will be transported to the surface. The number of confirmed surface polypeptides is relatively low in Campylobacter jejuni and includes mostly flagella structural proteins and a small number of non-flagella related surface proteins, such as PEB1-4.
The inventors have identified 51 different polypeptides in cell-surface fractions of Campylobacter jejuni. The method that was employed identifies polypeptides that are expressed at a relatively high level. The combination of being surface-exposed and being present in relatively high amounts makes these polypeptides highly suitable as targets for antibodies and thus for use in passive or active immunisation/vaccination. The invention only relates to the polypeptide defined by SEQ ID NO.43. The polypeptides defined by SEQ ID NOs 1-42, 44-51 are merely disclosed.

Fifteen of the 51 surface-located polypeptides that were identified (SEQ ID NO:37-51) have previously been described amongst more than 36,000 other loci as hits in homology searches using essential genes from other bacteria, see WO 02/077183. WO 02/077183 does not describe an identification of the subcellular localisation of the identified polypeptides nor a determination of their expression levels.

The combination of being surface-exposed and being present in relatively high amounts also makes the 51 polypeptides identified by the inventors highly suitable as targets for diagnosis of campylobacteriosis, allowing detection of intact cells with high sensitivity.

In addition, the surface-localisation of the 51 polypeptides makes them suitable as targets for inhibitors. Such inhibitors may be bactericidal or bacteristatic or prevent interaction of Campylobacter jejuni with the host organism (virulence).

### Definitions

- Vaccine - is used to indicate a composition capable of inducing a protective immune response against a microorganism in a human being or animal.
- Protective immune response - is used to indicate an immune response (humoral/antibody and/or cellular) inducing memory in an organism, resulting in the infectious agent, herein Campylobacter jejuni, being met by a secondary rather than a primary response, thus reducing its impact on the host organism.
- Polypeptide - unless specified otherwise, the term 'polypeptide' when used herein can also refer to a variant or fragment of a polypeptide. Preferred polypeptides are antigenic polypeptides.
- Fragment - is used to indicate a non-full length part of a polypeptide. Thus, a fragment is itself also a polypeptide.
- Variant - a 'variant' of a given reference polypeptide refers to a polypeptide that displays a certain degree of sequence identity to said reference polypeptide but is not identical to said reference polypeptide.
- Antigen / antigenic / antigenicity / immunogen / immunogenic / immunogenicity - all refer to the capability of inducing an immune response.
- Immunogenic carrier - refers to a compound which directly or indirectly assists or strengthens an immune response.
- Expression vector - refers to a, preferably recombinant, plasmid or phage or virus to be used in production of a polypeptide from a polynucleotide sequence. An expression vector comprises an expression construct, comprising an assembly of (1) a genetic element or elements having a regulatory role in gene expression, for example, promoters or enhancers, (2) a structural or coding sequence which is transcribed into mRNA and translated into protein, and which is operably linked to the elements of (1); and (3) appropriate transcription initiation and termination sequences.
- Binding partner - of a polypeptide refers to a molecule that can bind to said polypeptide. Such binding can be indirect, through another molecule, but is preferably direct. A binding partner can be any type of molecule, such as e.g. small hydrophobic molecules or e.g. a cellular or extracellular macromolecule, such as a protein, a carbohydrate or a nucleic acid. Preferred types of binding partners include antibodies, ligands or inhibitors.
- Plurality - the term 'plurality' indicates more than one, preferably more than 10.
- Indicator moiety - the term 'indicator moiety' covers a molecule or a complex of molecules that is capable of specifically binding a given polypeptide and/or cell, and is capable of generating a detectable signal. Preferably, the indicator moiety is an antibody or comprises an antibody molecule. Thus, a preferred indicator moiety is an antibody coupled to or in complex with a detectable substance.
- Host-derived molecule or host molecule - refers to a molecule which is normally found in a host organism that can be infected with *C. jejuni.* A host-derived molecule is preferably a host polypeptide, preferably a human polypeptide.
- Antibody - the term 'antibodies' when used herein is intended to cover antibodies as well as functional equivalents thereof. Thus, this includes polyclonal antibodies, monoclonal antibodies (mAbs), humanised, human or chimeric antibodies, single-chain antibodies, and also binding fragments of antibodies, such as, but not limited to, Fab fragments, F(ab')₂ fragments, fragments produced by a Fab expression library, anti-idiotypic antibodies, hybrids comprising antibody fragments, and epitope-binding fragments of any of the these. The term also includes multivalent, multispecific, such as bispecific antibodies and mixtures of monoclonal antibodies.
- Dissociation constant, Kd, is a measure to describe the strength of binding (or affinity or avidity) between macromolecules, for example an antibody and its antigen. The smaller Kd the stronger binding.
- Isolated - used in connection with polypeptides, polynucleotides and antibodies disclosed herein 'isolated' refers to these having been identified and separated and/or recovered from a component of their natural, typically cellular, environment. Contaminant components of the natural environment are materials that would typically interfere with diagnostic or therapeutic uses for the polypeptide, and may include enzymes, hormones, and other proteinaceous or nonproteinaceous solutes. Polypeptides, polynucleotides and antibodies of the invention are preferably isolated, and vaccines and other compositions of the invention preferably comprise isolated polypeptides or isolated polynucleotides or isolated antibodies.

### Detailed description

### Figures

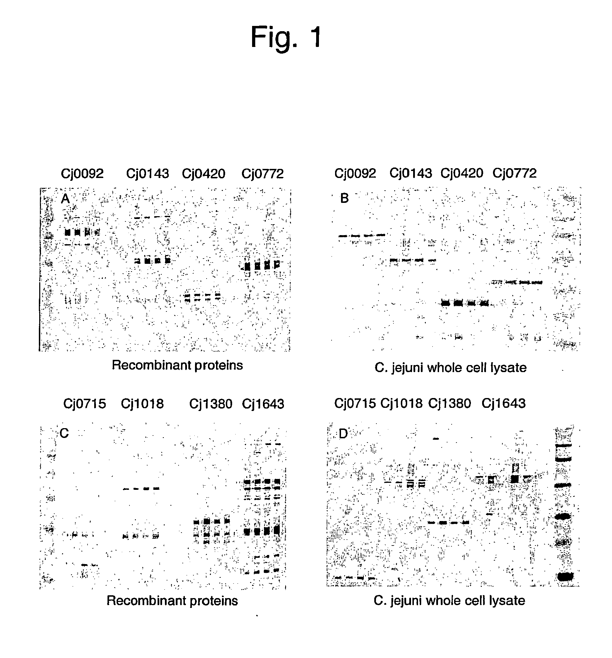

Figure 1. Sera from four randomly selected mice per antigen from experiments ACE003b and ACE003c were diluted 1:2000 and tested on Western blot against whole cell campylobacter lysate (B, D) or 100 ng of recombinant protein (A, C). Molecular weight marker proteins MW in kD: 97, 64, 51, 39, 28, 19, 14.

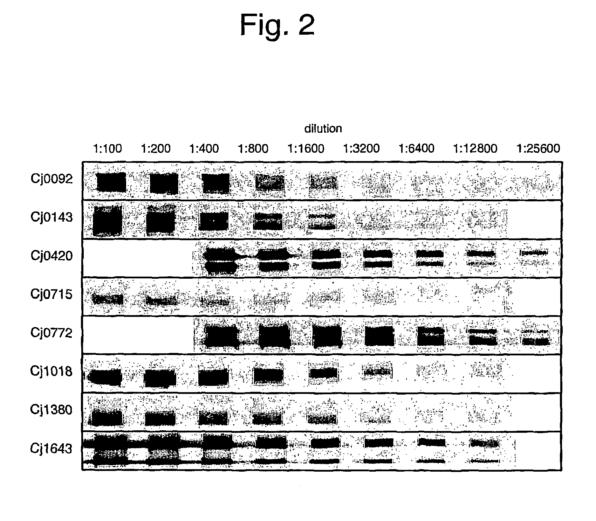

Figure 2. An example of antibody titer determination. Serum from a randomly selected mouse (one per antigen) from experiments ACE003b and ACE003c taken on day 21 (preceded by immunization on days 1 and 14) was serially diluted and Western blot was performed on corresponding recombinant protein (100ng/spot).

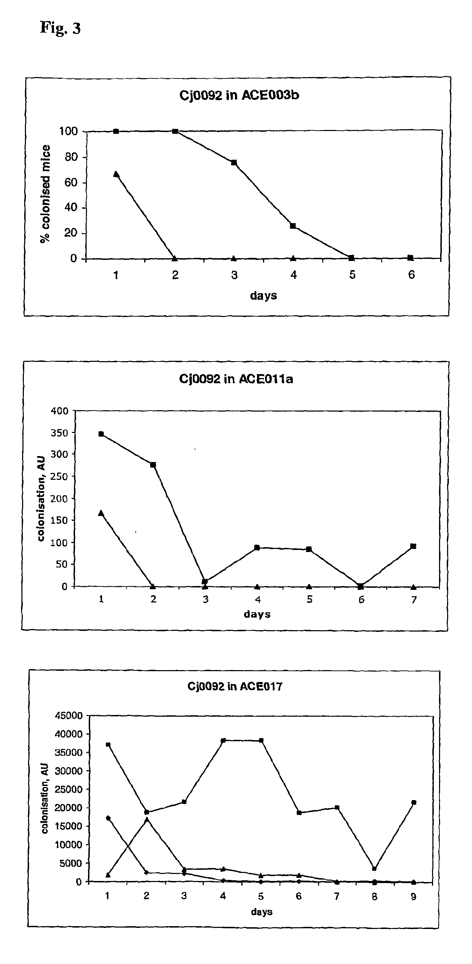

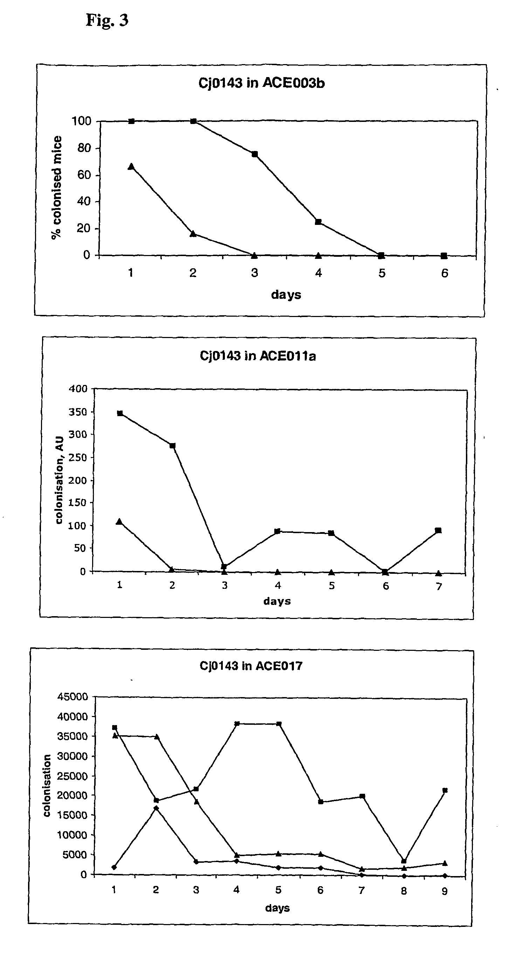

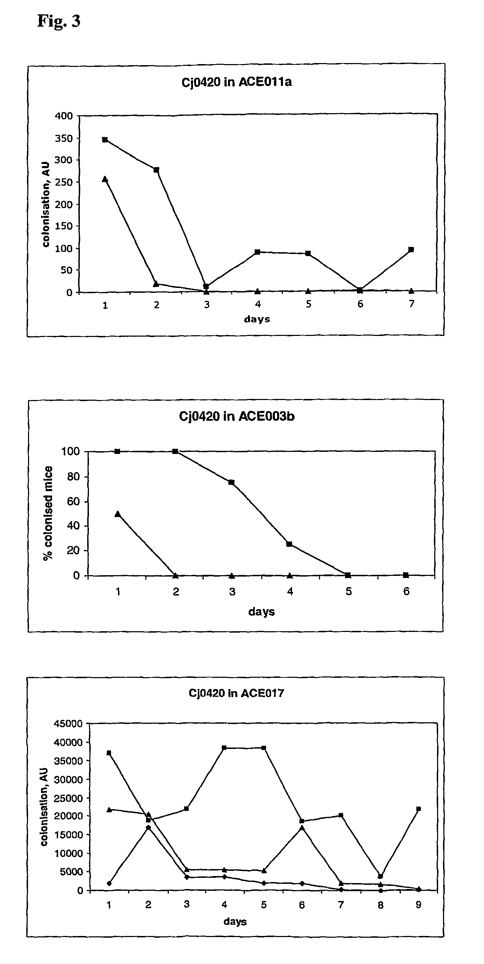

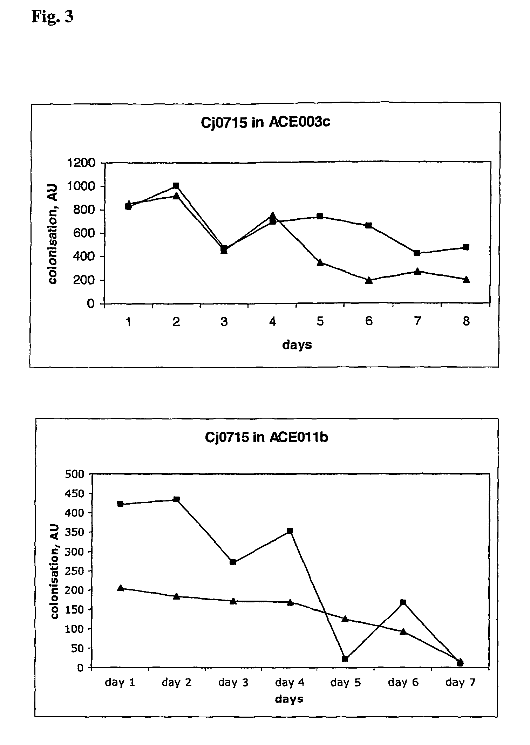

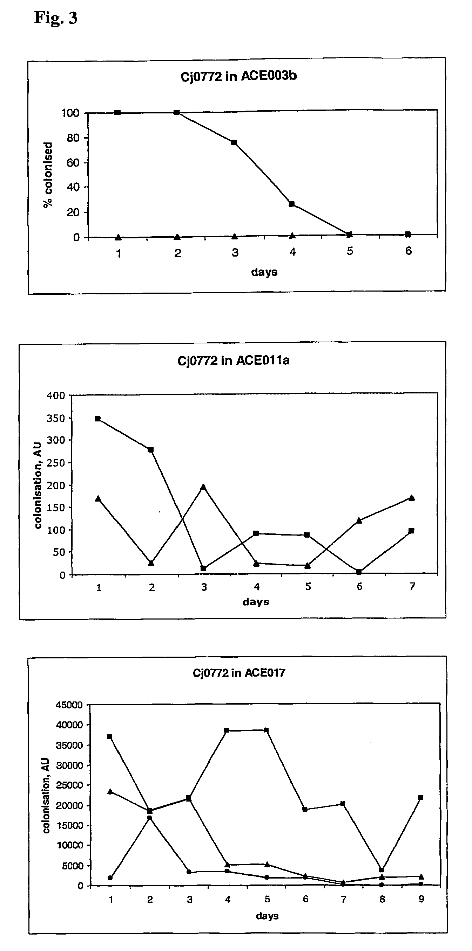

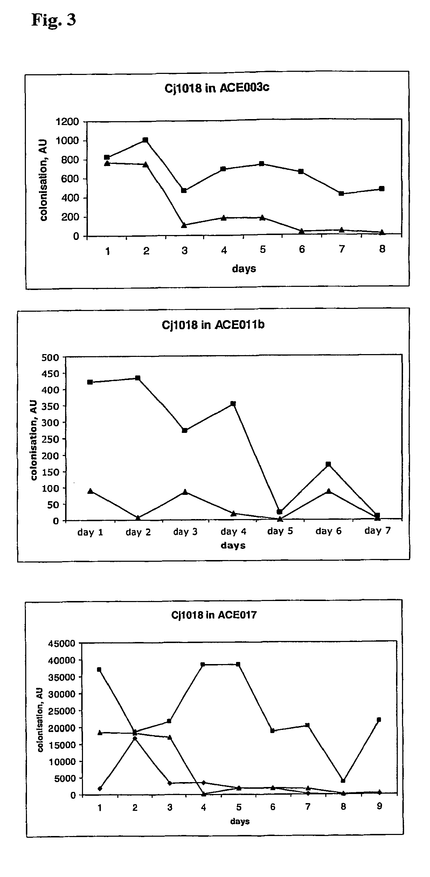

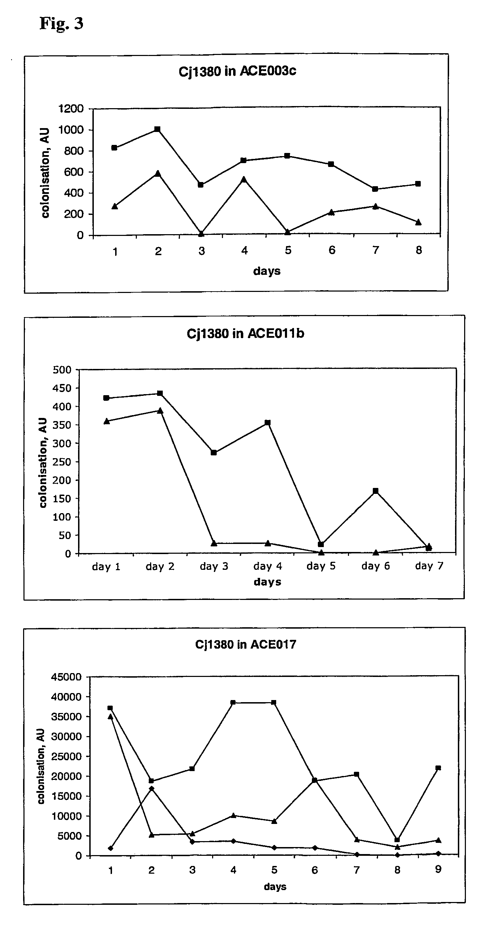

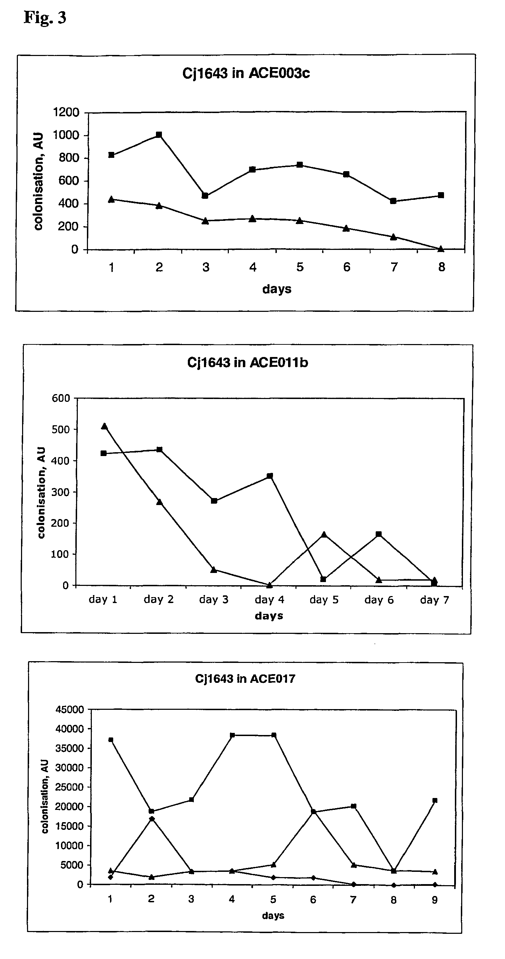

Figure 3. Results of mouse oral challenge. Five experiments including different combinations of antigens and controls were performed as listed below:
Alum negative control in squares, antigen in triangles, except for ACE017, there the antigen is also in triangles, but the positive control is glycine eluate (Rombus) and the negative control is CBP (see below) and is resembled by squares.

| Experiment name | Antigens used |
|---|---|
| ACE003b | Cj0092, Cj0143c, Cj0420, Cj0772c, alum |
| ACE003c | Cj0715, Cj1018c, Cj1380, Cj1643, alum |
| ACE011a | Cj0092, Cj0143c, Cj0420, Cj0772c, alum |
| ACE011b | Cj0715, Cj1018c, Cj1380, Cj1643, alum |
| ACE017 | Cj0092, Cj0143c, Cj0420, Cj0772c, Cj1018c, Cj1380, Cj1643, cellulose-binding domain from Clostridium cellulovorans (CBP, Sigma Aldrich C8581), glycine eluate from C.j. strains mentioned in the text. |

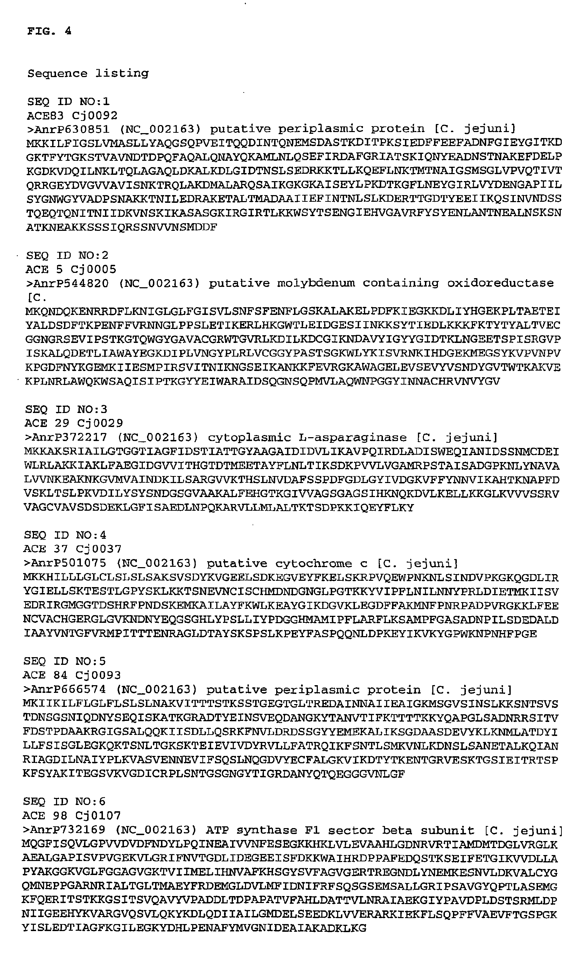

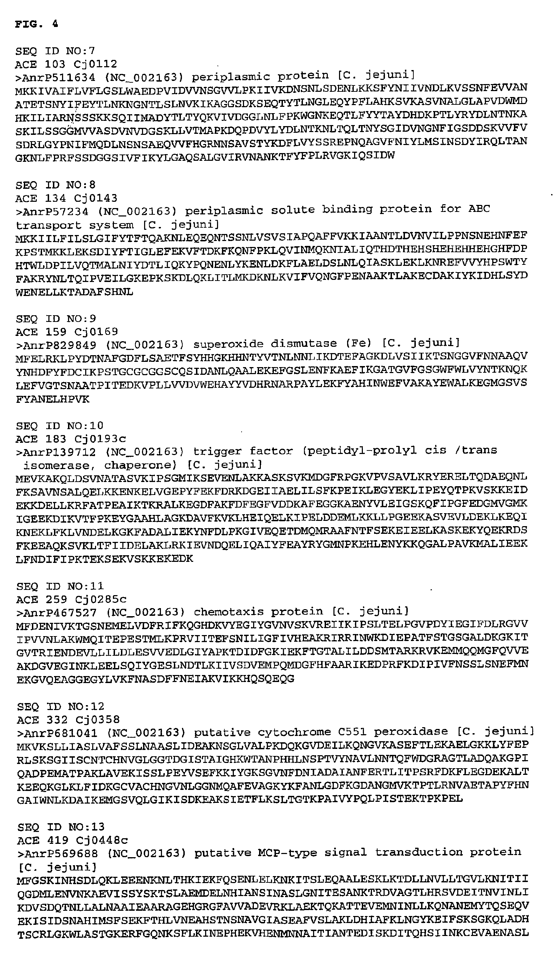

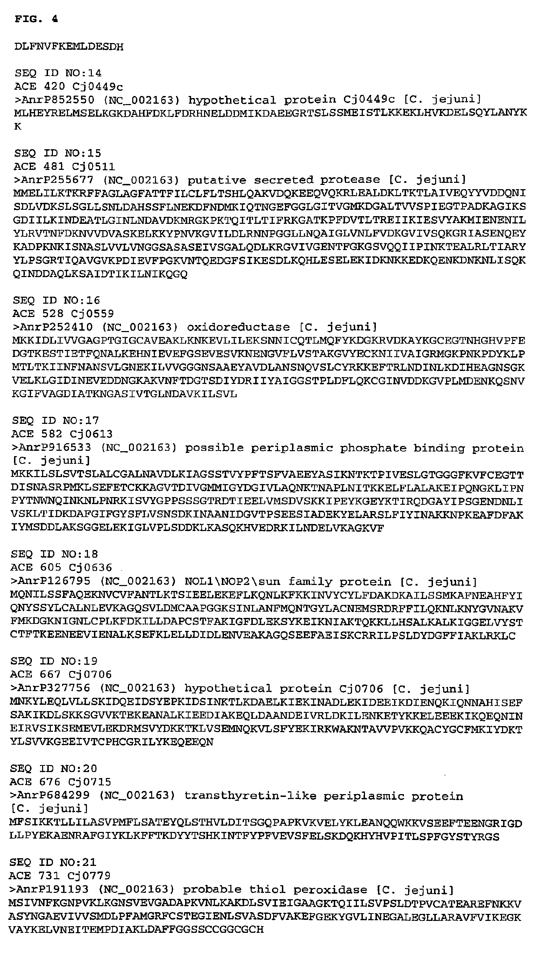

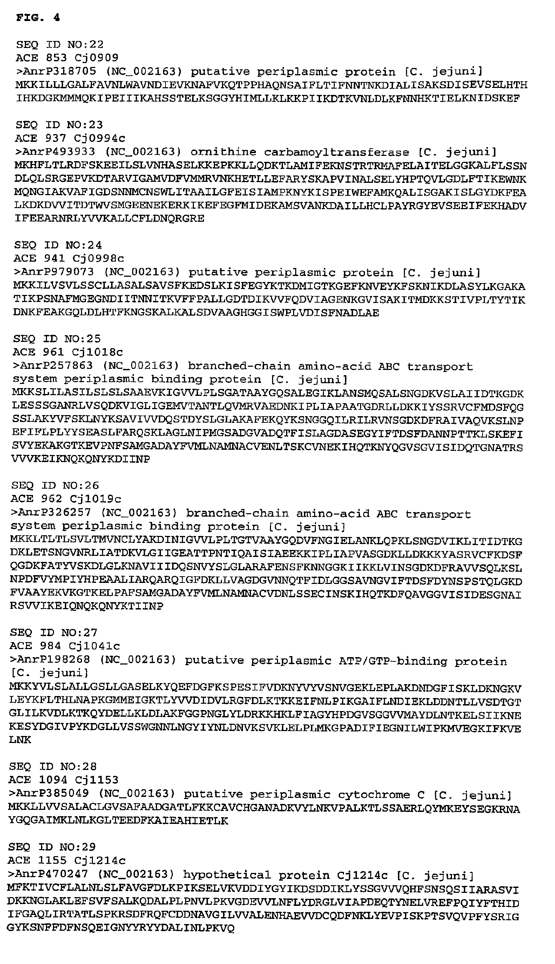

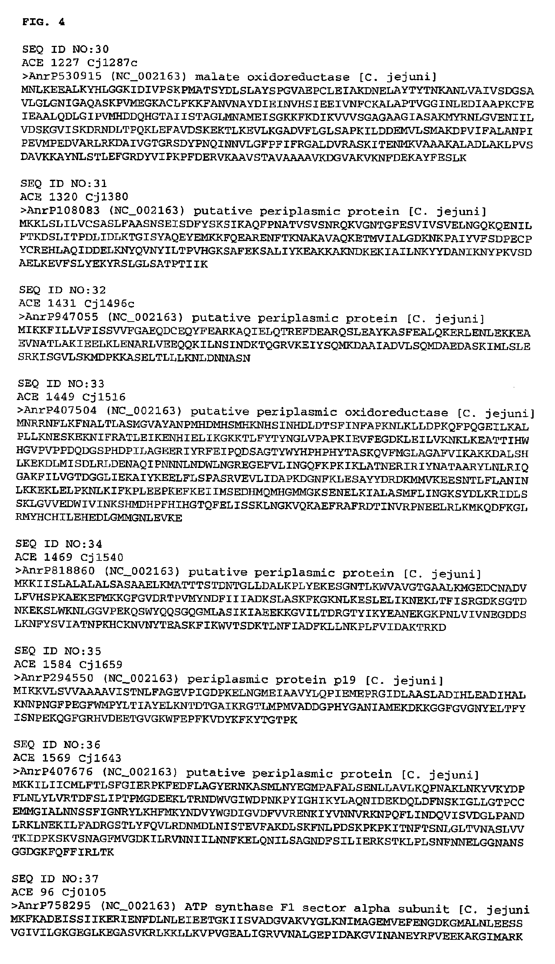

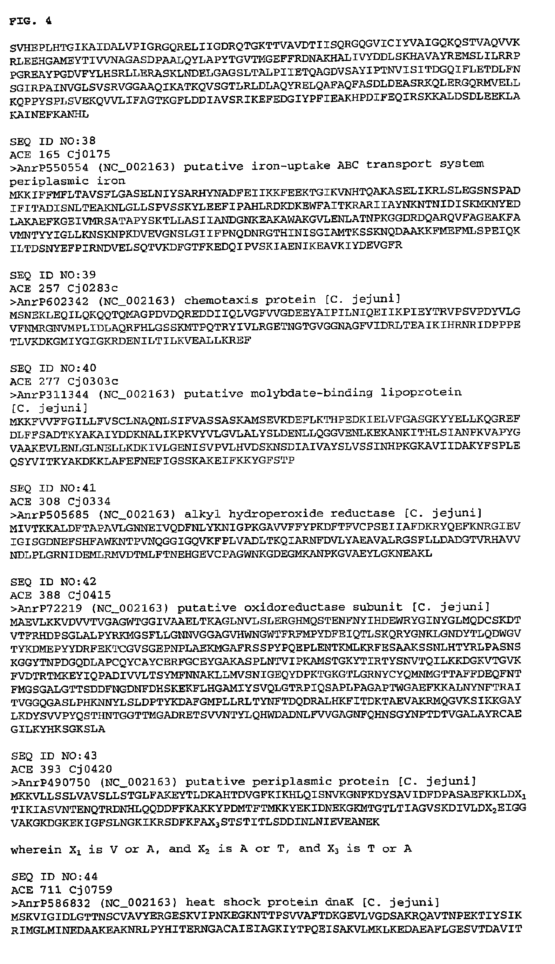

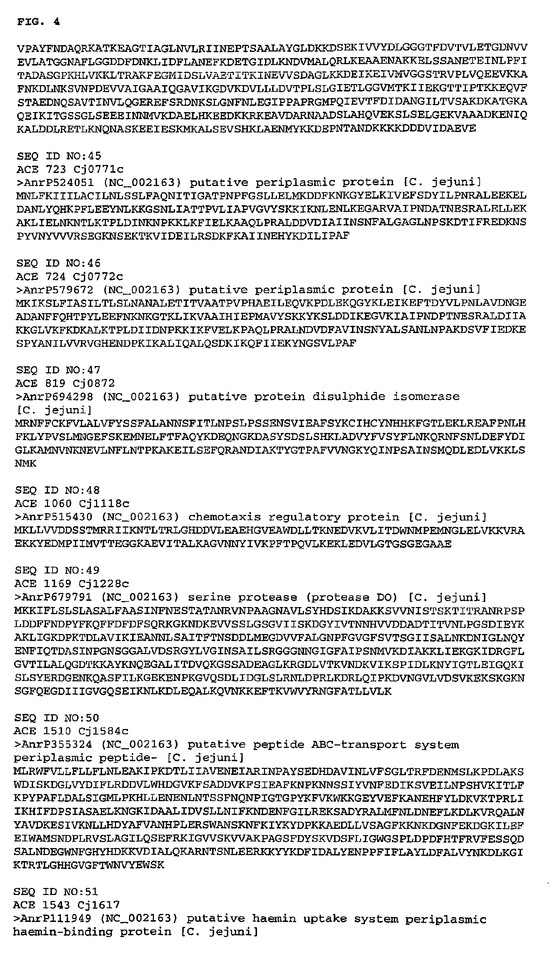

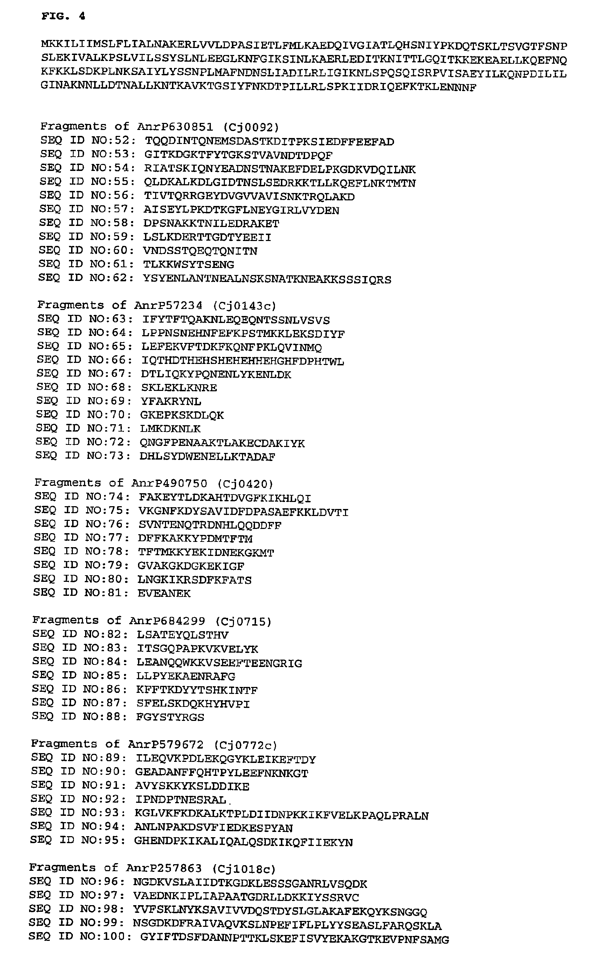

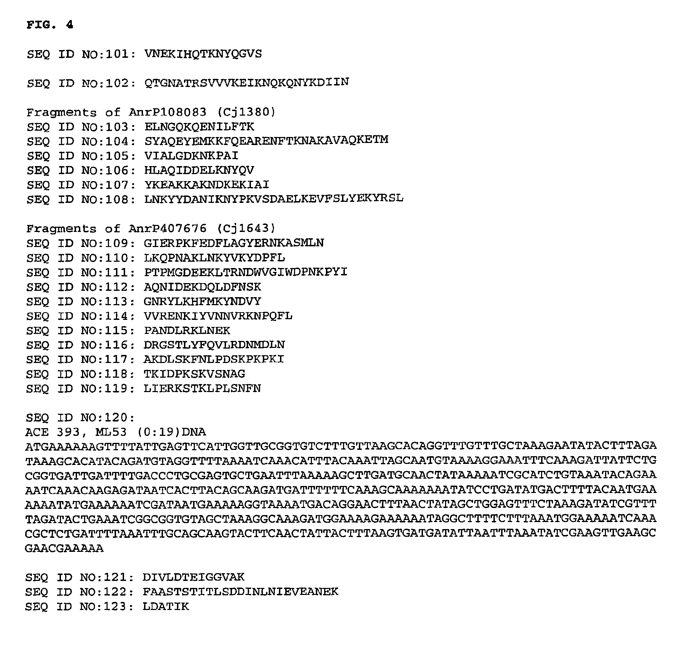

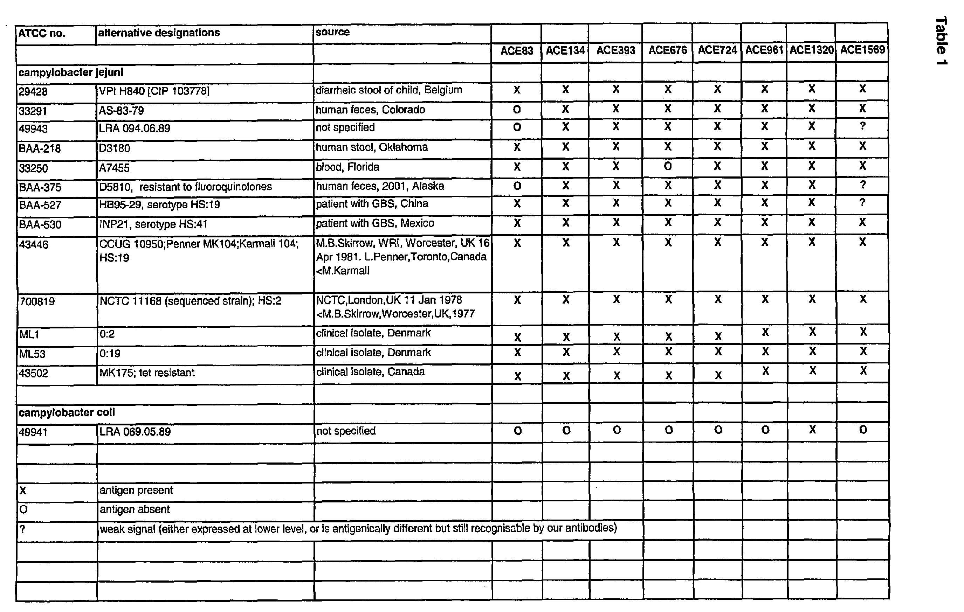

Figure 4. shows the sequence listing of the present application.

Table 1. Occurrence of 8 antigens in 13 randomly selected Campylobacter jejuni and one Campylobacter coli strain. Whole cell bacterial cell lysate from 14 strains listed was Western blotted and probed with sera against all 8 antigens.

### Vaccine compositions and methods of vaccination of the invention

The goal of vaccination or active immunisation is to provide protective immunity by inducing a memory response to an infectious microorganism using an antigenic or immunogenic composition. Thus, a vaccine is a composition capable of inducing a protective immune response against a microorganism in a human being or animal. Such an immune response can be a cellular response and/or a humoral response, e.g. a specific T cell response or an antibody response.

Accordingly, in an important embodiment, the composition is a vaccine composition. I.e. the invention relates to the use of a composition as defined in claim 1 as a vaccine.

The variant herein preferably has at least 95% sequence identity, such as at least 96%, e.g. at least 97%, such as at least 98%, e.g. at least 99% sequence identity to said sequence.

In another embodiment of the above composition, the polypeptide comprises SEQ ID NO:43, or an antigenic fragment or variant thereof. In one preferred embodiment, X₁ of SEQ ID NO:43 is V and X₂ is A and X₃ is T. In another preferred embodiment, X₁ of SEQ ID NO:43 is A and X₂ is T and X₃ is A.

The composition may only comprise one polypeptide defined by SEQ ID NO:43 or a fragment or variant thereof. However, in other embodiments, the composition also comprises other polypeptides of the group of SEQ ID NO:1-51 and/or fragments thereof. Thus, the composition according to the invention may also comprise more than one, such as 2, for example 3, such as 4, for example 5, such as 6, for example 7, such as 8, for example 9, such as 10, such as a number of polypeptides and/or fragments in the range of from 5 to 10, or more than 10, such as for example in the range of from 10 to 20, different polypeptides selected from the group of SEQ ID NO:1-51 or antigenic fragments or variants thereof.
Similarly, the composition may only comprise one polynucleotide, one expression vector or one recombinant virus or recombinant cell of the invention. However, in other embodiments, the composition comprises more than one polynucleotide, one expression vector or one recombinant virus or recombinant cell. Thus, the composition according to the invention may comprise more than one, such as 2, for example 3, such as 4, for example 5, such as 6, for example 7, such as 8, for example 9, such as 10, or more than 10, such as for example in the range of from 10 to 20, different polynucleotides, expression vectors or recombinant viruses or recombinant cells as described herein.
Furthermore, in some embodiments, a recombinant cell of the invention may express more than one polypeptide of the group of SEQ ID NO:1-51 and/or more than one antigenic fragment or variant of a polypeptide selected from the group of SEQ ID NO:1-51. Thus, the composition according to the invention may comprise a recombinant cell comprising more than one, such as 2, for example 3, such as 4, for example 5, such as 6, for example 7, such as 8, for example 9, such as 10, such as a number of polypeptides and/or antigenic fragments or variants in the range of from 5 to 10, or more than 10, such as for example in the range of from 10 to 20, different polypeptides selected from the group of SEQ ID NO:1-51 or antigenic fragments or variants thereof. In another embodiment, the composition for use in the invention comprises multiple of the recombinant viruses or recombinant cells described herein.

### Vaccines comprising polypeptides

In a preferred embodiment, the invention relates to a composition defined in claim 1 for use as a vaccine.

Accordingly, in this embodiment, the antigenicity or immunogenicity is provided by direct administration of a polypeptide normally located on the surface of a Campylobacter jejuni cell. In one particular embodiment, the polypeptides are selected so that the vaccine composition comprises multiple polypeptides capable of associating with different MHC molecules, such as different MHC class I molecules. Preferably, the composition for use as a vaccine comprises polypeptides and/or fragments capable of associating with the most frequently occurring MHC class I molecules. In one particular embodiment of the invention, the composition comprises one or more polypeptides and/or fragments capable of associating to an MHC class I molecule and one or more polypeptides and/or fragments capable of associating with an MHC class II molecule. Hence, the vaccine composition is in some embodiments capable of raising a specific cytotoxic T-cells response and/or a specific helper T-cell response. Association to MHC molecules can e.g. be determined as described by Andersen et al. (1999) Tissue Antigens 54:185; or by Tan et al. (1997) J. Immunol. Methods 209:25.

### Adjuvants and immunogenic carriers

Preferably, the composition for use as vaccine, i.e. the vaccine composition, of the present invention comprises a pharmaceutically-acceptable carrier as described herein in the section 'Compositions for use in the invention'.
The composition can further comprise an adjuvant. Adjuvants are substances whose admixture into the vaccine composition increases or otherwise modifies the immune response to a polypeptide or other antigen. Adjuvants could for example be any of: AlK(SO₄)₂, AlNa(SO₄)₂, AlNH₄(SO₄), silica, alum, Al(OH)₃, Ca₃ (PO₄)₂, kaolin, carbon, aluminium hydroxide, aluminium phosphate, muramyl dipeptides, N-acetyl-muramyl-L-threonyl-D-isoglutamine (thr-DMP), N-acetyl-nornuramyl-L-alanyl-D-iso-glutamine (CGP 11687, also referred to as nor-MDP), N-acetylmuramyul-L-alanyl-D-isoglutaminyl-L-alanine-2-(1'2'-dipalmitoyl-sn-glycero-3-hydroxphosphoryloxy)-ethylamine (CGP 19835A, also referred to as MTP-PE), RIBI (MPL+TDM+CWS) in a 2% squalene/Tween-80.RTM. emulsion, lipopolysaccharides and derivatives, including lipid A, Freund's Complete Adjuvant (FCA), Freund's Incomplete Adjuvants, Merck Adjuvant 65, polynucleotides (for example, poly IC and poly AU acids), wax D from Mycobacterium, tuberculosis, substances found in Corynebacterium parvum, Bordetella pertussis, and members of the genus Brucella, liposomes or other lipid emulsions, Titermax, ISCOMS, Quil A, ALUN (see US 58767 and 5,554,372), Lipid A derivatives, choleratoxin derivatives, HSP derivatives, LPS derivatives, synthetic peptide matrixes or GMDP, Interleukin 1, Interleukin 2, Montanide ISA-51 and QS-21. Preferred adjuvants to be used with the invention include Montanide ISA-51 and QS-21. Montanide ISA-51 (Seppic, Inc.) is a mineral oil-based adjuvant analogous to incomplete Freund's adjuvant, which is normally administered as an emulsion. QS-21 (Antigenics; Aquila Biopharmaceuticals, Framingham, MA) is a highly purified, water-soluble saponin that handles as an aqueous solution.
Desirable functionalities of adjuvants capable of being used in accordance with the present invention are listed in the below table.

**Table 1 Modes of adjuvant action**

| Action | Adjuvant type | Benefit |
|---|---|---|
| 1. Immunomodulation | Generally small molecules or proteins which modify the cytokine network | Upregulation of immune response. Selection of Th1 or Th2 |
| 2. Presentation | Generally amphipathio molecules or complexes which interact with immunogen in its native conformation | increased neutralizing antibody response. Greater duration of response |
| 3. CTL induction | • Particles which can bind or enclose immunogen and which can fuse with or disrupt cell membranes | Cytosolic processing of protein yielding correct class 1 restricted peptides |
| | • w/o emulsions for direct attachment of peptide to cell surface MHC-1 + | Simple process if promiscuous peptide(s) known |
| 4. Targeting | • Particulate adjuvants which bind immunogen. Adjuvants which saturate Kupffer cells | Efficient use of adjuvant and immunogen |
| | • Carbohydrate adjuvants which target lectin receptors on macrophages and DCs | As above. May also determine type of response if targeting selective |
| 5. Depot generation | • w/o emulsion for short term | Efficiency |
| | • Microspheres or nanospheres for long term | Potential for single-dose vaccine |

| | | |
|---|---|---|
| Source: John C. Cox and Alan R. Coulter Vaccine 1997 Feb;15(3):248-56 | | |

A vaccine composition according to the present invention may comprise more than one different adjuvant. It is also contemplated that the Campylobacter polypeptide of the invention, or one or more fragments thereof, and the adjuvant can be administered separately in any appropriate sequence.
Frequently, the adjuvant of choice is Freund's complete or incomplete adjuvant, or killed *B. pertussis* organisms, used e.g. in combination with alum precipitated antigen. A general discussion of adjuvants is provided in Goding, Monoclonal Antibodies: Principles & Practice (2nd edition, 1986) at pages 61-63. Goding notes, however, that when the antigen of interest is of low molecular weight, or is poorly immunogenic, coupling to an immunogenic carrier is recommended (see below). Various saponin extracts and cytokines have also been suggested to be useful as adjuvants in immunogenic compositions. Recently, it has been proposed to use granulocyte-macrophage colony stimulating factor (GM-CSF), a well known cytokine, as an adjuvant (WO 97/28816).
In addition, a vaccine composition of the invention can comprise an immunogenic carrier such as a scaffold structure, for example a protein or a polysaccharide, to which the Campylobacter polypeptide or the fragment thereof is capable of being associated. A Campylobacter polypeptide, or the antigenic fragment or variant thereof, present in the vaccine composition can be associated with an immunogenic carrier such as e.g. a protein. The binding or association of the polypeptide to a carrier protein may be covalent or non-covalent. An immunogenic carrier protein may be present independently of an adjuvant. The function of a carrier protein can for example be to increase the molecular weight of in particular fragments in order to increase their activity or immunogenicity, to confer stability, to increase the biological activity, or to increase serum half-life. Furthermore, an immunogenic carrier protein may aid presenting the Campylobacter polypeptide or the fragments thereof to T-cells. A carrier protein could be, but is not limited to, keyhole limpet hemocyanin, serum proteins such as transferrin, bovine serum albumin, human serum albumin, thyroglobulin or ovalbumin, immunoglobulins, or hormones, such as insulin. Tetanus toxoid and/or diptheria toxoid are also suitable carriers in one embodiment of the invention. Alternatively or additionally, dextrans, for example sepharose may be added. In yet another embodiment, an antigenpresenting cell such as e.g. a dendritic cell capable of presenting the polypeptide or a fragment thereof to a T-cell may be added to obtain the same effect as a carrier protein.

Methods for the preparation of vaccine compositions have e.g. been described in US 5,470,958 and references therein. An effective amount of the polypeptide component of a vaccine composition of the invention, if injected, will typically be in the range of from about 0.1 to about 1000 µg, such as e.g. from about 1 to about 900 µg, for example from about 5 to about 500 µg, for a human subject, and generally in the range of from about 0.01 to 10.0 µg/Kg body weight of a subject animal. The above-indicated ranges are merely indicative and should not be interpreted as limiting the present invention.
An effective amount of an antigenic polypeptide of the invention may be an amount capable of eliciting a detectable humoral immune response in the absence of an immunomodulator. For many immunogens, this is in the range of about 5-100 µg for a human subject. The appropriate amount of immunogen to be used is dependent on the immunological response it is desired to elicit. Furthermore, the exact effective amount necessary will vary from subject to subject, depending on the species, age and general condition of the subject, the severity of the condition being treated, the mode of administration, etc. It is therefore not always possible to specify an exact effective amount. However, the appropriate effective amount may be determined by one of ordinary skill in the art using only routine experimentation or prior knowledge in the art.

### DNA vaccine compositions and vaccine compositions comprising recombinant viruses or recombinant cells

DNA or RNA vaccines pertain to the introduction of e.g. an antigenic polypeptide determinant into a patient by overexpressing in the cells of the patient, a polynucleotide construct which includes expression control sequences operably linked to a sequence encoding the polypeptide of interest, herein a polypeptide of SEQ ID NO:43 or an antigenic fragment or variant thereof as defined in claim 1. As such fragments may not contain a methionine start codon, such a codon is optionally included as part of the expression control sequences. The polynucleotide construct may be a nonreplicating and linear polynucleotide, a circular expression vector, or an autonomously replicating plasmid or viral expression vector. The construct may become integrated into the host genome. Any expression vector that can transfect a mammalian cell may be used in the methods of immunising an individual according to the present invention. Methods for constructing expression vectors are well known in the art (see, e.g., Molecular Cloning: A Laboratory Manual, Sambrook et al., eds., Cold Spring Harbor Laboratory, 2nd Edition, Cold Spring Harbor, N.Y., 1989). Preferred are compositions comprising a plurality of genes expressing multiple polypeptides selected from SEQ ID NO:1-51 and/or multiple antigenic fragments of the invention, thereby permitting simultaneous vaccination against a variety of preselected targets.
Vaccines can also be prepared by incorporating a polynucleotide encoding a specific antigenic polypeptide of interest into a living but harmless vector, such as a virus or a cell, such as an attenuated or reduced-virulence E. coli or Salmonella cell. The harmless recombinant virus or recombinant cell is injected into the intended recipient. Such a recombinant cell may be dead or alive. If alive, the recombinant organism may replicate in the host while producing and presenting the antigenic polypeptide to the host's immune system. It is contemplated that this type of vaccine may be more effective than the non-replicative type of vaccine. For such a vaccine to be successful, the vector organism must be viable, and either be naturally non-virulent or have an attenuated or reduced-virulence phenotype.
Strategies for vaccination using attenuated bacteria and suitable bacterial strains for use therein have been described in e.g. Makino et al. (2001) Microb. Pathog. 31:1-8; Gentschev et al. (2002) Int. J. Med. Microbiol. 291:577-582; Turner et al. (2001) Infect. Immun. 69:4969-4979; WO99/49026; and WO03/022307.
Further examples of vectors that can be applied are vectors comprising e.g., retroviruses, as disclosed in WO 90/07936, WO 91/02805, WO 93/25234, WO 93/25698, and WO 94/03622, adenovirus, as disclosed by Berkner, Biotechniques 6:616-627, 1988; Li et al., Hum. Gene Ther. 4:403-409, 1993; Vincent et al., Nat. Genet. 5:130-134, 1993; and Kolls et al., Proc. Natl. Acad. Sci. USA 91:215-219, 1994), pox virus, as disclosed by U.S. 4,769,330; U.S. Pat. No. 5,017,487; and WO 89/01973, naked DNA as disclosed WO 90/11092, a polynucleotide molecule complexed to a polycationic molecule as disclosed in WO 93/03709, and polynucleotides associated with liposomes as disclosed by Wang et al., Proc. Natl. Acad. Sci. USA 84:7851, 1987. In certain embodiments, the DNA may be linked to killed or inactivated adenovirus as disclosed by Curiel et al., Hum. Gene Ther. 3:147-154, 1992; Cotton et al., Proc. Natl. Acad. Sci. USA 89:6094,1992. Other suitable compositions include DNA-ligands as disclosed by Wu et al., J. Biol. Chem. 264:16985-16987, 1989), and lipid-DNA combinations as disclosed by Felgner et al., Proc. Natl. Acad. Sci. USA 84:7413-7417, 1989). In addition, the efficiency of naked DNA uptake into cells may be increased by coating the DNA onto biodegradable latex beads.
Vaccine vectors preferably comprise a suitable promoter which is operably linked to the polynucleotide sequence encoding the immunogenic polypeptide. Any promoter that can direct a high level of transcription initiation in the target cells may be used in the invention. Non-tissue specific promoters, such as the cytomegalovirus (DeBernardi et al., Proc Natl Acad Sci USA 88:9257-9261 [1991], and references therein), mouse metallothionine I (Hammer et al., J Mol Appl Gen 1:273-288 [1982]), HSV thymidine kinase (McKnight, Cell 31:355-365 [1982]), and SV40 early (Benoist et al., Nature 290:304-310 [1981]) promoters may thus also be used.

### Methods of vaccination

In a further main aspect, the present invention relates to the use of a composition defined in claim 1 for the preparation of a medicament for the immunisation of an animal or human being against Campylobacter jejuni infections. The immunisation preferably induces a protective immune response.

The animal may be any bird or mammal, e.g. a chicken, duck, turkey, cow or pig. Particular target populations of human beings may be individuals from at-risk populations, such as the population of children up to 4 years old, the population of persons in industrialised nations or the population of naive or semi-immune travellers to the developing world.
Modes of administration of the composition according to the invention include, but are not limited to systemic administration, such as intravenous or subcutaneous administration, intradermal administration, intramuscular administration, intranasal administration, oral administration, and generally any form of mucosal administration.

The immunogenic effect according to the present invention can e.g. be measured by assay of antibodies in serum samples e.g. by a RIA. Furthermore, the effect can be determined in vivo, by measuring e.g. an increased T cell responsiveness to T cell dependent antigenic polypeptides, wherein said increased responsiveness is characteristic of an enhancement of a normal immune response to such antigenic polypeptides. An immunostimulating effect may also be measured as an enhanced T cell production of, in particular, IL-2, IL-3, IFN-γ and/or GM-CSF. Polypeptides or fragments thereof having a potential for eliciting an enhanced immune response may thus be readily identified by screening for enhanced IL-2, IL-3, IFN-γ or GM-CSF production by T cells. Young et al. (2000) In: Campylobacter, 2nd ed. Ed. by Nachamkin and Blaser, American Society for Microbiology, pp. 287-301 also describe a series of suitable animal models which can be of use in the evaluation of the efficacy of therapeutic and preventive strategies and compositions. A number of aspects related to vaccination against Campylobacter, including potential target populations, animal models and vaccination strategies have been described by Scott and Tribble (2000) In: Campylobacter, 2nd ed. Ed. by Nachamkin and Blaser, American Society for Microbiology, pp. 303-319).
The herein described polynucleotides and expression vectors can be introduced into target cells in vivo or in vitro by any standard method: e.g., as naked DNA (Donnelly et al., Annu Rev Immunol 15:617-648 [1997]), incorporated into ISCOMS, liposomes, or erythrocyte ghosts, or by biolistic transfer, calcium precipitation, or electroporation. Alternatively, one can employ a viral-based vector as a means for introducing the polynucleotide encoding the polypeptide of interest into the cells of the animal or human being. Preferred viral vectors include those derived from replication-defective hepatitis viruses (e.g., HBV and HCV), retroviruses (see, e.g., WO89/07136; and Rosenberg et al., N Eng J Med 323 (9):570-578 [1990]), adenovirus (see, e.g., Morsey et al., J Cell Biochem, Supp. 17E [1993]), adenoassociated virus (Kotin et al., Proc Natl Acad Sci USA 87:2211-2215 [1990]), replication defective herpes simplex viruses (HSV; Lu et al., Abstract, page 66, Abstracts of the Meeting on Gene Therapy, Sep. 22-26, 1992, Cold Spring Harbor Laboratory, Cold Spring Harbor, N.Y.), canary pox virus, and any modified versions of these vectors. Cells transfected in vitro can be cultured and cloned, if desired, prior to introduction into the patient.
In addition to direct in vivo procedures, ex vivo procedures may be used in which cells are removed from an animal, modified, and placed into the same or another animal. It will be evident that one can utilise any of the compositions noted above for introduction of an antigenic polypeptides or polynucleotides encoding such according to the invention into tissue cells in an ex vivo context. Protocols for viral, physical and chemical methods of uptake are well known in the art. Thus, as an alternative to administration of a polypeptide of the invention or a vector capable of expressing such a polypeptide directly to the patient, one can remove helper T cells from the patient; stimulate those T cells ex vivo using the same antigenic polypeptide or vector; and introduce the stimulated helper T cells into the same patient.

### Antibodies and methods for raising antibodies of the invention

In a further main embodiment, the composition for use as a medicament comprises an antibody capable of binding a polypeptide defined by SEQ ID NO:43. Such a medicament can be used for antibody therapy, such as passive immunisation of an individual in need thereof.
In another main aspect, the invention relates to an antibody capable of binding, preferably specifically binding, an intact Campylobacter jejuni cell and capable of binding, preferably specifically binding, a polypeptide selected from the polypeptide of SEQ ID NO:43 and/or a fragment and/or a variant thereof as defined in claim 1.

Preferred antibodies are ones that bind with a dissociation constant or Kd of less than 5 X 10⁻⁶M, such as less than 10⁻⁶M, e.g. less than 5 X 10⁻⁷M, such as less than 10⁻⁷M, e.g. less than 5 X 10⁻⁸M, such as less than 10⁻⁸M, e.g. less than 5 X 10⁻⁹M, such as less than 10⁻⁹M, e.g. less than 5 X 10⁻¹⁰M, such as less than 10⁻¹⁰M, e.g. less than 5 X 10⁻¹¹M, such as less than 10⁻¹¹M, e.g. less than 5 X 10⁻¹²M, such as less than 10⁻¹²M, e.g. less than 5 X 10⁻¹³M, such as less than 10⁻¹³M, e.g. less than 5 X 10⁻¹⁴M, such as less than 10⁻¹⁴M, e.g. less than 5 X 10⁻¹⁵M, or less than 10⁻¹⁵M. Binding constants can be determined using methods well-known in the art, such as ELISA (e.g. as described in Orosz and Ovadi (2002) J. Immunol. Methods 270:155-162) or surface plasmon resonance analysis.

Antibodies can be used for passive immunisation of mammals, preferably human beings, more preferably immunocompromised patients. A treatment with antibodies can be done to cure or to prevent Campylobacter jejuni infections.
Antibodies of the invention include the following preferred mechanistic groups:
1. Function-inhibiting antibodies that work as an antibacterial (affect the viability of the bacterium). Such antibodies should be effective regardless of the immune status of the patient. Preferably, such antibodies are capable of reducing Campylobacter jejuni growth in vitro to less than 50%, such as less than 25%, for example less than 10%, such as less than 5% of a control without antibody added.
2. Opsonising antibodies that are designed to enhance phagocytic killing. Effectiveness of such antibodies may depend on the immune status of the patient, but it is very well possible that they will enhance phagocytic killing even in compromised patients.
3. Antibodies conjugated to a therapeutic moiety such as a toxin or bactericidal agent, e g. ricin or radioisotopes. Techniques for conjugating a therapeutic moiety to antibodies are well known, see, e.g. Thorpe et al.(1982) Immunol. Rev. 62, 119-158. These antibodies should also be effective regardless of the immune status of the patient.

An antibody with or without a therapeutic moiety conjugated to it can be used as a therapeutic that is administered alone or in combination with chemotherapeutics or other therapeutic agents.

In one embodiment, the antibodies of the invention are opsonising as well as function-inhibiting. In another embodiment, the antibodies of the invention are opsonising but not function-inhibiting. The latter group of antibodies can e.g. be antibodies directed against a target polypeptide which is not essential for the viability of Campylobacter.

In another main aspect, the invention discloses to a method for raising antibodies to a polypeptide defined by SEQ ID NO:43 in an non-human animal, wherein the antibodies are capable of binding an intact Campylobacter jejuni cell, the method comprising the steps of
a. providing
   - a polypeptide consisting of SEQ ID NO:43, or comprising antigenic fragment or variant of said sequence as defined in claim 1,
   - a polynucleotide comprising a sequence encoding said polypeptide,
   - an expression vector comprising a sequence encoding said polypeptide, or
   - a recombinant virus or recombinant cell comprising said polynucleotide or said expression vector,
b. introducing a composition comprising said polypeptide, polynucleotide, vector, recombinant virus or recombinant cell into said animal,
c. raising antibodies in said animal,
d. isolating and optionally purifying the antibodies, and
e. selecting antibodies capable of binding an intact Campylobacter jejuni cell.

The above methods are preferably done in a transgenic animal which is capable of producing human antibodies. In a further preferred embodiment, the above methods are non-therapeutic.

### Monoclonal/polyclonal antibodies

Antibodies of the invention may be polyclonal antibodies or monoclonal antibodies or mixtures of monoclonal antibodies. In a preferred embodiment, the antibody is a monoclonal antibody or a fragment thereof. Monoclonal antibodies (Mab's) are antibodies wherein every antibody molecule is similar and thus recognises the same epitope. The antibody may be any kind of antibody, however, it is preferably an IgG or IgA antibody.
Monoclonal antibodies are in general produced by a hybridoma cell line. Methods of making monoclonal antibodies and antibody-synthesising hybridoma cells are well known to those skilled in the art. Antibody-producing hybridomas may for example be prepared by fusion of an antibody-producing B lymphocyte with an immortalised cell line. A monoclonal antibody can be produced by the following steps. An animal is immunised with an antigen such as a full-length polypeptide or a fragment thereof. The immunisation is typically accomplished by administering the antigen to an immunologically competent mammal in an immunologically effective amount, i.e., an amount sufficient to produce an immune response. Preferably, the mammal is a rodent such as a rabbit, rat or mouse. The mammal is then maintained on a booster schedule for a time period sufficient for the mammal to generate high affinity antibody molecules. A suspension of antibody-producing cells is removed from each immunised mammal secreting the desired antibody. After a sufficient time to generate high affinity antibodies, the animal (e.g. mouse) is sacrificed and antibody-producing lymphocytes are obtained from one or more of the lymph nodes, spleens and peripheral blood. Spleen cells are preferred, and can be mechanically separated into individual cells in a physiological medium using methods well known to one of skill in the art. The antibody-producing cells are immortalised by fusion to cells of a mouse myeloma line. Mouse lymphocytes give a high percentage of stable fusions with mouse homologous myelomas, however, rat, rabbit and frog somatic cells can also be used. Spleen cells of the desired antibody-producing animals are immortalised by fusing with myeloma cells, generally in the presence of a fusing agent such as polyethylene glycol. Any of a number of myeloma cell lines suitable as a fusion partner can be, for example, the P3-NS1/1-Ag4-1, P3-x63-Ag8.653 or Sp2/O-Ag14 myeloma lines, available from the American Type Culture Collection (ATCC), Rockville, Md.
Monoclonal antibodies can also be generated by other methods well known to those skilled in the art of recombinant DNA technology. An alternative method, referred to as the "combinatorial antibody display" method, has been developed to identify and isolate antibody fragments having a particular specificity, and can be utilised to produce monoclonal antibodies.
A polyclonal antibody is a mixture of antibody molecules recognising a specific given antigen, hence polyclonal antibodies may recognise different epitopes within e.g. a polypeptide. In general polyclonal antibodies are purified from serum of a mammal, which previously has been immunised with the antigen. Polyclonal antibodies may for example be prepared by any of the methods described in Antibodies: A Laboratory Manual, By Ed Harlow and David Lane, Cold Spring Harbor Laboratory Press, 1988*.* Polyclonal antibodies may be derived from any suitable mammalian species, for example from mice, rats, rabbits, donkeys, goats, and sheep.

### Specificity

The antibodies of the invention may be monospecific towards the polypeptides of SEQ ID NO:43. In another embodiment, the antibody is bispecific or multispecific having at least one portion being also specific towards any of the polypeptides of SEQ ID NO:1-51.
Monospecific antibodies may be monovalent, i.e. having only one binding domain. For a monovalent antibody, the immunoglobulin constant domain amino-acid sequences preferably comprise the structural portions of an antibody molecule known in the art as CH1, CH2, CH3 and CH4. Preferred are those which are known in the art as C_{L}. Furthermore, insofar as the constant domain can be either a heavy or light chain constant domain (C_{H} or C_{L}, respectively), a variety of monovalent antibody compositions are contemplated by the present invention. For example, light chain constant domains are capable of disulphide bridging to either another light chain constant domain, or to a heavy chain constant domain. In contrast, a heavy chain constant domain can form two independent disulphide bridges, allowing for the possibility of bridging to both another heavy chain and to a light chain, or to form polymers of heavy chains. Thus, in another embodiment, the invention contemplates a composition comprising a monovalent polypeptide wherein the constant chain domain C has a cysteine residue capable of forming at least one disulphide bridge, and where the composition comprises at least two monovalent polypeptides covalently linked by said disulphide bridge.
In another embodiment of the invention the antibody is a multivalent antibody having at least two binding domains. The binding domains may have specificity for the same ligand or for different ligands.

### Multispecificity, including bispecificity

In a preferred embodiment the invention relates to multispecific antibodies, which have affinity for and are capable of specifically binding at least two different entities.
In one embodiment, the multispecific antibody is a bispecific antibody, which carries at least two different binding domains, at least one of which is of antibody origin. A bispecific molecule of the invention can also be a single chain bispecific molecule. Multispecific molecules can also be single-chain molecules or may comprise at least two single-chain molecules. The multispecific, including bispecific antibodies, may be produced by any suitable manner known to the person skilled in the art. A number of approaches have been developed such as the ones described in WO 94/09131; WO 94/13804; WO 94/13806 or U.S. Pat. Nos. 5,260,203; 5,455,030; 4,881,175; 5,132,405; 5,091,513; 5,476,786; 5,013,653; 5,258,498; and 5,482,858.

Using a bispecific or multispecific antibody according to the invention the invention offers several advantages as compared to monospecific/monovalent antibodies. A bispecific/multispecific antibody has a first binding domain capable of specifically recognising and binding any of the Campylobacter jejuni polypeptides of SEQ ID NO:1-51, whereas the other binding domain(s) may be used for other purposes. In one embodiment, at least one other binding domain is used for binding to a Campylobacter jejuni polypeptide, such as binding to another epitope on the same Campylobacter jejuni polypeptide as the first binding domain. Thereby specificity for Campylobacter jejuni may be increased as well as increase of avidity of the antibody. In another embodiment the at least one other binding domain may be used for specifically binding a mammalian cell, such as a human cell. It is preferred that the at least other binding domain is capable of binding an immunoactive cell, such as a leukocyte, a macrophage, a lymphocyte, a basophilic cell, and/or an eosinophilic cell, in order to increase the effect of the antibody in a therapeutic method. This may be accomplished by establishing that the at least one other binding domain is capable of specifically binding a mammalian protein, such as a human protein, such as a protein selected from any of the cluster differentiation proteins (CD), in particular CD64 and/or CD89.

### Humanised antibodies

It is not always desirable to use non-human antibodies for human therapy, since the non-human "foreign" epitopes may elicit an immune response in the individual to be treated. To eliminate or minimise the problems associated with non-human antibodies, it is desirable to engineer chimeric antibody derivatives, i.e., "humanised" antibody molecules that combine the non-human Fab variable region binding determinants with a human constant region (Fc). Such antibodies are characterised by equivalent antigen specificity and affinity of the monoclonal and polyclonal antibodies described above, and are less immunogenic when administered to humans, and therefore more likely to be tolerated by the individual to be treated.
Accordingly, in one embodiment the antibody of the invention is a humanised antibody. Humanised antibodies are in general chimeric antibodies comprising regions derived from a human antibody and regions derived from a non-human antibody, such as a rodent antibody. Humanisation (also called Reshaping or CDR-grafting) is a well-established technique for reducing the immunogenicity of monoclonal antibodies (mAbs) from xenogeneic sources (commonly rodent), increasing the homology to a human immunoglobulin, and for improving their activation of the human immune system. Thus, humanised antibodies are typically human antibodies in which some CDR residues and possibly some framework residues are substituted by residues from analogous sites in rodent antibodies.
It is important that humanised antibodies retain high affinity for the antigen and other favourable biological properties. To achieve this goal, according to a preferred method, humanised antibodies are prepared by a process of analysis of the parental sequences and various conceptual humanised products using three-dimensional models of the parental and humanised sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of certain residues in the functioning of the candidate immunoglobulin sequence, i.e., the analysis of residues that influence the ability of the candidate immunoglobulin to bind its antigen. In this way, FR residues can be selected and combined from the recipient and import sequences so that the desired antibody characteristic, such as increased affinity for the target antigen(s), is maximised, although it is the CDR residues that directly and most substantially influence antigen binding.
One method for humanising MAbs relates to production of chimeric antibodies in which an antigen binding site comprising the complete variable domains of one antibody is fused to constant domains derived from a second antibody, preferably a human antibody. Methods for carrying out such chimerisation procedures are for example described in EP-A-0 120 694 (Celltech Limited), EP-A-0 125 023 (Genentech Inc.), EP-A-0 171 496 (Res. Dev. Corp. Japan), EP-A-0173494 (Stanford University) and EP-A-0 194 276 (Celltech Limited).
The humanised antibody of the present invention may be made by any method capable of replacing at least a portion of a CDR of a human antibody with a CDR derived from a non-human antibody. Winter describes a method which may be used to prepare the humanised antibodies of the present invention (UK Patent Application GB 2188638A).
As an example, the humanised antibodies of the present invention may be produced by the following process:
(a) constructing, by conventional techniques, an expression vector containing an operon with a DNA sequence encoding an antibody heavy chain in which the CDRs and such minimal portions of the variable domain framework region that are required to retain antibody binding specificity are derived from a non-human immunoglobulin, and the remaining parts of the antibody chain are derived from a human immunoglobulin;
(b) constructing, by conventional techniques, an expression vector containing an operon with a DNA sequence encoding a complementary antibody light chain in which the CDRs and such minimal portions of the variable domain framework region that are required to retain donor antibody binding specificity are derived from a non-human immunoglobulin, and the remaining parts of the antibody chain are derived from a human immunoglobulin;
(c) transfecting the expression vectors into a host cell by conventional techniques; and
(d) culturing the transfected cell by conventional techniques to produce the humanised antibody.

The host cell may be co-transfected with the two vectors of the invention, the first vector containing an operon encoding a light chain derived polypeptide and the second vector containing an operon encoding a heavy chain derived polypeptide. The two vectors contain different selectable markers, but otherwise, apart from the antibody heavy and light chain coding sequences, are preferably identical, to ensure, as far as possible, equal expression of the heavy and light chain polypeptides. Alternatively, a single vector may be used, the vector including the sequences encoding both the light and the heavy chain polypeptides. The coding sequences for the light and heavy chains may comprise cDNA or genomic DNA or both.
The host cell used to express the altered antibody of the invention may be either a bacterial cell such as Escherichia coli, or a eukaryotic cell. In particular a mammalian cell of a well defined type for this purpose, such as a myeloma cell or a Chinese hamster ovary cell may be used.
The general methods by which the vectors of the invention may be constructed, transfection methods required to produce the host cell of the invention and culture methods required to produce the antibody of the invention from such host cells are all conventional techniques. Likewise, once produced, the humanised antibodies of the invention may be purified according to standard procedures.

### Human antibodies

In a more preferred embodiment the invention relates to an antibody, wherein the binding domain is carried by a human antibody, i.e. wherein the antibodies have a greater degree of human peptide sequences than do humanised antibodies.
Human mAb antibodies directed against human proteins can be generated using transgenic mice carrying the human immune system rather than the mouse system. Splenocytes from these transgenic mice immunised with the antigen of interest are used to produce hybridomas that secrete human mAbs with specific affinities for epitopes from a human protein (see, e.g., Wood et al. International Application WO 91/00906, Kucherlapati et al. PCT publication WO 91/10741; Lonberg et al. International Application WO 92/03918; Kay et al. International Application 92/03917; Lonberg, N. et al. 1994 Nature 368:856-859; Green, L. L. et al. 1994 Nature Genet. 7:13-21; Morrison, S. L. et al. 1994 Proc. Natl. Acad. Sci. USA 81:6851-6855; Bruggeman et al. 1993 Year Immunol 7:33-40; Tuaillon et al. 1993 PNAS 90:3720-3724; Bruggeman et al. 1991 Eur J Immunol 21:1323-1326). Such transgenic mice are available from Abgenix, Inc., Fremont, Calif., and Medarex, Inc., Annandale, N.J. It has been described that the homozygous deletion of the antibody heavy-chain joining region (IH) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, e.g., Jakobovits et al., Proc. Natl. Acad. Sci. USA 90:2551 (1993); Jakobovits et al., Nature 362:255-258 (1993); Bruggemann et al., Year in Immunol. 7:33 (1993); and Duchosal et al. Nature 355:258 (1992). Human antibodies can also be derived from phage-display libraries (Hoogenboom et al ., J. Mol. Biol. 227: 381 (1992); Marks et al., J. Mol. Biol. 222:581-597 (1991); Vaughan, et al., Nature Biotech 14:309 (1996)).
Suitable methods for producing human monoclonal antibodies have furthermore been described in WO 03/017935, WO 02/100348, US 2003 091561, and US 2003 194403.

### Binding fragments of antibodies

In one embodiment of the invention, the antibody is a fragment of an antibody, preferably an antigen binding fragment or a variable region. Examples of antibody fragments useful with the present invention include Fab, Fab', F(ab')₂ and Fv. Papain digestion of antibodies produces two identical antigen binding fragments, called the Fab fragment, each with a single antigen binding site, and a residual "Fc" fragment, so-called for its ability to crystallise readily. Pepsin treatment yields an F(ab')₂ fragment that has two antigen binding fragments which are capable of cross-linking antigen, and a residual other fragment (which is termed pFc'). Additional fragments can include diabodies, linear antibodies, single-chain antibody molecules, and multispecific antibodies formed from antibody fragments.
The antibody fragments Fab, Fv and scFv differ from whole antibodies in that the antibody fragments carry only a single antigen-binding site. Recombinant fragments with two binding sites have been made in several ways, for example, by chemical cross-linking of cysteine residues introduced at the C-terminus of the VH of an Fv (Cumber et al., 1992), or at the C-terminus of the VL of an scFv (Pack and Pluckthun, 1992), or through the hinge cysteine residues of Fab's (Carter et al., 1992).
Preferred antibody fragments retain some or essentially all of the ability of an antibody to selectively binding with its antigen. Some preferred fragments are defined as follows:
(1) Fab is the fragment that contains a monovalent antigen-binding fragment of an antibody molecule. A Fab fragment can be produced by digestion of whole antibody with the enzyme papain to yield an intact light chain and a portion of one heavy chain.
(2) Fab' is the fragment of an antibody molecule and can be obtained by treating whole antibody with pepsin, followed by reduction, to yield an intact light chain and a portion of the heavy chain. Two Fab' fragments are obtained per antibody molecule. Fab' fragments differ from Fab fragments by the addition of a few residues at the carboxyl terminus of the heavy chain CH1 domain including one or more cysteines from the antibody hinge region.
(3) (Fab')₂ is the fragment of an antibody that can be obtained by treating whole antibody with the enzyme pepsin without subsequent reduction. F(ab')₂ is a dimer of two Fab' fragments held together by two disulfide bonds.
(4) Fv is the minimum antibody fragment that contains a complete antigen recognition and binding site. This region consists of a dimer of one heavy and one light chain variable domain in a tight, non-covalent association (V_{H} -V_{L} dimer). It is in this configuration that the three CDRs of each variable domain interact to define an antigen binding site on the surface of the V_{H} -V_{L} dimer. Collectively, the six CDRs confer antigen binding specificity to the antibody. However, even a single variable domain (or half of an Fv comprising only three CDRs specific for an antigen) has the ability to recognise and bind antigen, although at a lower affinity than the entire binding site.

In one embodiment of the present invention the antibody is a single-chain antibody, defined as a genetically engineered molecule containing the variable region of the light chain, the variable region of the heavy chain, linked by a suitable polypeptide linker as a genetically fused single chain molecule. Such single-chain antibodies are also referred to as "single-chain Fv" or "sFv" antibody fragments. Generally, the Fv polypeptide further comprises a polypeptide linker between the V_{H} and V_{L} domains that enables the sFv to form the desired structure for antigen binding.
The antibody fragments according to the invention may be produced in any suitable manner known to the person skilled in the art. Several microbial expression systems have already been developed for producing active antibody fragments, e.g. the production of Fab in various hosts, such as E. coli or yeast has been described. The fragments can be produced as Fab's or as Fv's, but additionally it has been shown that a V_{H} and a V_{L} can be genetically linked in either order by a flexible polypeptide linker, which combination is known as an scFv.

### Compositions for use in the invention

In a preferred embodiment of the composition for use as a medicament, said composition comprises, in addition to the active component, a pharmaceutically-acceptable carrier.

As used herein, the term "pharmaceutically acceptable" used in connection with compositions or carriers represents that the materials are capable of being administered to or upon a human or animal without the production of undesirable physiological effects such as nausea, dizziness, gastric upset and the like.

The preparation of a composition that contains active ingredients dissolved or dispersed therein is well understood in the art. Often such compositions are prepared as sterile injectables either as liquid solutions or suspensions, aqueous or nonaqueous, however, solid forms suitable for solution, or suspension, in liquid prior to use can also be prepared. The preparation can also be emulsified. The active ingredient can be mixed with carriers which are pharmaceutically acceptable and compatible with the active ingredient and in amounts suitable for use in the methods described herein. Suitable carriers are, for example, water, saline, dextrose, glycerol, ethanol or the like and combinations thereof. In addition, if desired, the composition can contain minor amounts of auxiliary substances such as wetting or emulsifying agents, pH buffering agents and the like which enhance the effectiveness of the active ingredient.

The compositions of the present invention can include pharmaceutically-acceptable salts of the components therein. Pharmaceutically acceptable salts include the acid addition salts (formed with the free amino groups of the polypeptide) that are formed with inorganic acids such as, for example, hydrochloric or phosphoric acids, or such organic acids as acetic, tartaric, mandelic and the like. Salts formed with the free carboxyl groups can also be derived from inorganic bases such as, for example, sodium, potassium, ammonium, calcium or ferric hydroxides, and such organic bases as isopropylamine, trimethylamine, 2-ethylamino ethanol, histidine, procaine and the like.
Pharmaceutically-acceptable carriers are well known in the art. Exemplary of liquid carriers are sterile aqueous solutions that contain no materials in addition to the active ingredients and water, or contain a buffer such as sodium phosphate at physiological pH value, physiological saline or both, such as phosphate-buffered saline. Still further, aqueous carriers can contain more than one buffer salt, as well as salts such as sodium and potassium chlorides, dextrose, propylene glycol, polyethylene glycol and other solutes. Liquid compositions can also contain liquid phases in addition to and to the exclusion of water. Exemplary of such additional liquid phases are glycerin, vegetable oils such as cottonseed oil, organic esters such as ethyl oleate, and water-oil emulsions.
A composition containing a polypeptide or antibody of the present invention preferably contains an amount of at least 0.1 weight percent of polypeptide or antibody per weight of total pharmaceutical composition. A weight percent is a ratio by weight of polypeptide or antibody to total composition. Thus, for example, 0.1 weight percent is 0.1 grams of polypeptide or antibody per 100 grams of total composition.

The composition may also be a kit-in-part further including an antibiotic agent, such as antibiotics selected from β-lactams, cephalosporins, penicilins, aminoglycosides, macrolide antibiotics (erythromycin, clarithromycin, or azithromycin) and fluoroquinolone antibiotics (ciprofloxacin, levofloxacin, gatifloxacin, or moxifloxacin) and/or including an immunostimulating agent, such as cytokines, interferons, growth factors, for example GCSF or GM-CSF. The kit-in-part may be used for simultaneous, sequential or separate administration.

### Polypeptides of the invention

### Fragments of the invention

In a further aspect, the invention relates to a fragment of SEQ ID NO:43 defined by any of SEQ ID NOs 74-81.

Preferably, fragments of the invention are surface-exposed in an intact Campylobacter jejuni cell or other cell when expressed recombinantly therein. Surface-exposure can be e.g. be determined using a monoclonal antibody specific for said fragment, e.g. as described in Singh et al. (2003) Infect. Immun. 71:3973-3946. Also preferred are fragments which are capable of inducing antibodies that can specifically bind an intact Campylobacter jejuni cell. This can be determined by generating monoclonal antibodies using said fragment and subsequent characterisation of the binding of individual antibodies to intact cells, e.g. as described in Singh et al. (2003) Infect. Immun. 71:3973-3946.

The full-length polypeptide of SEQ ID NO:43 as well as the fragments of the invention can be produced recombinantly by conventional techniques known in the art. Suitable host cells can be mammalian cells, e.g. CHO, COS or HEK293 cells. Alternatively, insect cells, bacterial cells or fungal cells can be used. Methods for heterologous expression of polynucleotide sequences in the cell types listed above and subsequent purification of the produced polypeptides, e.g. using a tag sequence such as a histidine tag, which may be removed after purification, are well-known to those skilled in the art. Alternatively, fragments of the invention can be produced synthetically.

### Variants of the invention

In a further main aspect, the invention relates to the use of variants of the polypeptide set forth in SEQ ID NO:43 as defined in claim 1 in a composition for use as a medicament.
When used herein, phrases such as 'a polypeptide having at least 95% sequence identity to SEQ ID NO:X' are used interchangeably with, and are intended to be directed to the same subject-matter as, phrases such as 'the polypeptide of SEQ ID NO:X and variants thereof, wherein the variant has at least 95% sequence identity to said sequence.'

Variants have at least 75% sequence identity, for example at least 80% sequence identity, such as at least 85% sequence identity, for example at least 90% sequence identity, such as at least 91% sequence identity, such as at least 92% sequence identity, for example at least 93% sequence identity, such as at least 94% sequence identity, for example at least 95% sequence identity, such as at least 96% sequence identity, for example at least 97% sequence identity, such as at least 98% sequence identity, for example 99% sequence identity with the given polypeptide or fragment. Sequence identity is determined with any of the algorithms GAP, BESTFIT, or FASTA in the Wisconsin Genetics Software Package Release 7.0, using default gap weights.

Preferred variants of a given polypeptide or fragment are variants in which all amino-acid substitutions between the variant and the given reference polypeptide or fragment are conservative substitutions. Conservative amino-acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine, a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfurcontaining side chains is cysteine and methionine. Preferred conservative aminoacids substitution groups are: valine-leucine-isoleucine, phenylalanine-tyrosine, lysine-arginine, alanine-valine, and asparagine-glutamine.
Variants of a polypeptide or of a fragment thereof also include forms of the polypeptide or fragment wherein one or more amino acids have been deleted or inserted. Preferably, less than 5, such as less than 4, e.g. less than 3, such as less than 2, e.g. only one amino acid has been inserted or deleted. 'Variants' of a polypeptide or of a fragment thereof also include forms of these polypeptides or fragments modified by post-translational modifications of the amino-acid sequence.

### Polynucleotides and expression vectors of the invention

Preferred expression vectors as ones suitable for DNA vaccination. Other preferred expression vectors are ones in which a polynucleotide of the invention is under the control of a promoter that directs expression of the sequence in Escherichia coli or Salmonella. The latter expression vectors are useful in the production of a recombinant virus or recombinant cell of the invention as described herein.
The polynucleotides and expression vectors of the invention can be prepared by standard recombinant DNA techniques well-known to the person skilled in the art.

### Recombinant cells of the invention

Suitable bacterial strains for use herein have been described in e.g. Makino et al. (2001) Microb. Pathog. 31:1-8; Gentschev et al. (2002) Int. J. Med. Microbiol. 291:577-582; Turner et al. (2001) Infect. Immun. 69:4969-4979; WO99/49026; and WO03/022307 and references therein. Examples of suitable Salmonella strains are CvD908-T7pol (Santiago-Machuca et al. (2002) Plasmid 47:108-119), ATCC 39183, ATCC 53647 and ATCC 53648. Examples of suitable E. coli strains are YT106 and E1392/75-2A.

### Methods and uses of the invention

The compositions and other products defined above can be used to treat or prevent Campylobacter jejuni infections, and/or disease resulting from such infections, in animals or human beings in need thereof. Preferably, the animal is a chicken, duck, turkey, cow or pig. Preferred human populations are at-risk populations, such as the population of children up to 4 years old, the population of persons in industrialised nations or the population of naive or semi-immune travellers to the developing world.

Treatment and prevention herein include all types of therapeutic treatment and preventive treatment and other treatments to combat Campylobacter jejuni, including but not limited to vaccination, prophylaxis, active immunisation, passive immunisation, administration of antibodies, curative treatment, ameliorating treatment. In particular, passive immunisation using antibodies of the invention is a suitable treatment for immunocompromised individuals.

Preferably, said administration is done parenterally, intravenously, intramuscularly, subcutaneously, orally or intranasally.

Preferably, said medicament is a medicament suitable for parenteral, intravenous, intramuscular, subcutaneous, oral or intranasal administration.

### Diagnostic methods of the invention

The combination of being surface-exposed and being present in relatively high copy numbers in cells also makes the 51 polypeptides identified by the inventors highly suitable as targets for detection of Campylobacter jejuni, allowing detection of this organism with high sensitivity.

In another aspect, the invention relates to a method for detecting a Campylobacter jejuni comprising the steps of
a. contacting said sample with an indicator moiety capable of specifically binding a polypeptide consisting of SEQ ID NO:43, wherein the indicator moiety furthermore is capable of binding, preferably specifically binding, intact Campylobacter jejuni cells, and
b. determining whether a signal has been generated by the indicator moiety, thereby detecting whether said sample contains Campylobacter jejuni or parts thereof.

In preferred embodiments of the above diagnostic methods, a washing step is performed between the contacting step and the determination step, in order to improve the specificity of detection.

The sample can e.g. be faeces, urine, a tissue, tissue extract, fluid sample or body fluid sample, such as blood, plasma or serum. Another example of a sample is a food sample, such as a meat sample.

The above methods can e.g. be used to diagnose Campylobacter jejuni infections or campylobacteriosis in an individual. In preferred embodiments of the above methods, said indicator moiety does not pass through the outer-membrane of a Campylobacter jejuni cell. A preferred type of said indicator moiety consists of or comprises an antibody, such as an antibody of the invention as defined herein.

Those skilled in the art will understand that there are numerous well known clinical diagnostic chemistry procedures in which an indicator moiety can be used to form an binding reaction product whose amount relates to the amount of the ligand, herein C. jejuni or parts thereof, in a sample. Thus, while exemplary assay methods are described herein, the invention is not so limited.

The present invention also relates to a diagnostic system, preferably in kit form, for assaying for the presence, and preferably also the amount, of Campylobacter jejuni in a biological sample. Methods for the preparation of diagnostic kits have e.g. been described in US 5,470,958 and references therein.
The diagnostic system includes, in an amount sufficient to perform at least one assay, an indicator moiety according to the present invention, preferably as a separately packaged reagent, and more preferably also instructions for use. Packaged refers to the use of a solid matrix or material such as glass, plastic (e.g., polyethylene, polypropylene or polycarbonate), paper, foil and the like capable of holding within fixed limits an indicator moiety of the present invention. Thus, for example, a package can be a glass vial used to contain milligram quantities of a contemplated labelled indicator moiety preparation, or it can be a microtiter plate well to which microgram quantities of a contemplated indicator moiety has been operatively affixed, i.e., linked so as to be capable of binding a ligand.
"Instructions for use" typically include a tangible expression describing the reagent concentration or at least one assay method parameter such as the relative amounts of reagent and sample to be admixed, maintenance time periods for reagent/sample admixtures, temperature, buffer conditions and the like.

In most embodiments, the diagnostic method and system of the present invention include as a part of the indicator moiety, a label or indicating means capable of signalling the formation of a binding reaction complex containing an indicator moiety complexed with the preselected ligand. Such labels are themselves well-known in clinical diagnostic chemistry.
The labelling means can be a fluorescent labelling agent that chemically binds to antibodies or antigens without denaturing them to form a fluorochrome (dye) that is a useful immunofluorescent tracer. Suitable fluorescent labelling agents are fluorochromes such as fluorescein isocyanate (FIC), fluorescein isothiocyante (FITC), 5-dimethylamine-1-naphthalenesulfonyl chloride (DANSC), tetramethylrhodamine isothiocyanate (TRITC), lissamine, rhodamine 8200 sulphonyl chloride (RB 200 SC). Other examples of suitable fluorescent materials include umbelliferone, dichlorotriazinylamine fluorescein, dansyl chloride or phycoerythrin and the like. A description of immunofluorescence analysis techniques is found in DeLuca, "Immunofluorescence Analysis", in Antibody As a Tool, Marchalonis, et al., eds., John Wiley & Sons, Ltd., pp. 189-231 (1982).
Radioactive elements can be useful as labelling agents. An exemplary radiolabeling agent is a radioactive element that produces gamma ray emissions. Elements which themselves emit gamma rays, such as ¹²⁴I, ¹²⁵I, ¹²⁸I, ¹³²I and ⁵¹Cr represent one class of gamma ray emission-producing radioactive element indicating groups. Particularly preferred is ¹²⁵I. Another group of useful labelling means are those elements such as ¹¹C, ¹⁸F, ¹⁵O and ¹³N which themselves emit positrons, or beta emitters, such as ¹¹¹indium of ³H. Other suitable radioactive materials include ¹³¹I and ³⁵S.
Detection using antibodies can, in other embodiments, be facilitated by coupling the antibody to another detectable substance, such as an enzyme, a prosthetic group, a luminescent materials, or a bioluminescent material. Examples of suitable enzymes include horseradish peroxidase, alkaline phosphatase, betagalactosidase, or acetylcholinesterase; examples of suitable prosthetic group complexes include Streptavidin/biotin and avidin/biotin; an example of a luminescent material includes luminol; examples of bioluminescent materials include luciferase, luciferin, and aequorin.
In preferred embodiments, the indicating group is an enzyme, such as horseradish peroxidase (HRP) or glucose oxidase. In such cases where the principal indicating group is an enzyme such as HRP or glucose oxidase, additional reagents are required to visualise the fact that a indicator-moiety/ligand complex (immunoreactant) has formed. Such additional reagents for HRP include hydrogen peroxide and an oxidation dye precursor such as diaminobenzidine. An additional reagent useful with glucose oxidase is 2,2'-amino-di-(3-ethyl-benzthiazoline-G-sulfonic acid).
The linking of labels, i.e. labelling of polypeptides such as antibodies, is well known in the art. For instance, proteins can be labelled by metabolic incorporation of radioisotope-containing amino acids provided as a component in the culture medium. See, for example, Galfre et al., Meth. Enzymol., 73:3-46 (1981). The techniques of protein conjugation or coupling through activated functional groups are particularly applicable. See, for example, Aurameas, et al., Scand. J. Immunol., Vol. 8 Suppl. 7:7-23 (1978), Rodwell et al. (1984) Biotech. 3:889-894, and U.S. Pat. No. 4,493,795.
Various diagnostic assays employing the above indicator moieties can be set up to test samples for *Campylobacfer jejuni*. Exemplary assays are described in detail in Antibodies: A Laboratory Manual, Harlow and Lane (eds.), Cold Spring Harbor Laboratory Press, 1988. Representative examples of such assays include: countercurrent immuno-electrophoresis (CIEP), radioimmunoassays, radioimmunoprecipitations, enzyme-linked immuno-sorbent assays (ELISA), dot blot assays, inhibition or competition assays, and sandwich assays, immunostick (dipstick) assays, simultaneous immunoassays, immunochromatographic assays, immunofiltration assays, latex bead agglutination assays, immunofluorescent assays, biosensor assays, and low-light detection assays (see e.g. also U.S. 4,376,110 and 4,486,530).
In one embodiment, the diagnostic kits of the present invention can be used in an "ELISA" format to detect the quantity of a preselected ligand in a fluid sample. "ELISA" refers to an enzyme-linked immunosorbent assay that employs an antibody or antigen bound to a solid phase and an enzyme-antigen or enzyme-antibody conjugate to detect and quantify the amount of an antigen present in a sample and is readily applicable to the present methods. Thus, in some embodiments, an indicator moiety of the present invention can be affixed to a solid matrix to form a solid support that comprises a package in the subject diagnostic systems. A reagent is typically affixed to a solid matrix by adsorption from an aqueous medium although other modes of affixation applicable to polypeptides, such as antibodies, can be used that are well known to those skilled in the art. Useful solid matrices are also well known in the art. Such materials are water insoluble and include the cross-linked dextran available under the trademark SEPHADEX from Pharmacia Fine Chemicals (Piscataway, N.J.); agarose; beads of polystyrene beads about 1 micron to about 5 millimetres in diameter available from Abbott Laboratories of North Chicago, Ill.; polyvinyl chloride, polystyrene, cross-linked polyacrylamide, nitrocellulose- or nylon-based webs such as sheets, strips or paddles; or tubes, plates or the wells of a microtiter plate such as those made from polystyrene or polyvinylchloride.
A further diagnostic method may utilise the multivalency of an antibody composition of one embodiment of this invention to cross-link ligands, thereby forming an aggregation of multiple ligands and polypeptides, producing a precipitable aggregate. This embodiment is comparable to the well known methods of immune precipitation. This embodiment comprises the steps of admixing a sample with a composition comprising an antibody of this invention to form a binding admixture under binding conditions, followed by a separation step to isolate the formed binding complexes. Typically, isolation is accomplished by centrifugation or filtration to remove the aggregate from the admixture. The presence of binding complexes indicates the presence of the preselected ligand to be detected.

### Binding partners and inhibitors of polypeptides of the invention

The surface-localisation of the 51 polypeptides which this invention discloses makes them highly suitable as targets for binding partners, such as inhibitors. Surface-located polypeptides of a pathogenic microorganism often interact with the host organism. Thus, any type of binding partner of a surface-located polypeptide may interfere with host-pathogen interaction. Binding partners thus often antagonise the pathogenicity (virulence) of a microorganism. A binding partner may also be an inhibitor of the polypeptide it binds.

Thus, in a further main aspect, the invention relates to methods for the identification of binding partners of the surface-located polypeptide set forth in SEQ ID NO:43. Such methods may be biochemical or cell-based.

### Biochemical methods

In one aspect, a host-derived binding partner of a polypeptide defined by SEQ ID NO:43, may be identified as follows: purified host membranes are electrophoretically separated, blotted over to a membrane and incubated with the polypeptide of interest or fragment thereof. Binding can then be detected using antibodies specific for the polypeptide of interest or fragment thereof. The host binding partner to which the polypeptide or fragment thereof has bound can subsequently be identified by elution from the blot and subsequent analysis by mass spectrometry, or by any other technique known in the art.
If the binding partner of a surface-located polypeptide of a pathogenic organism is a host-derived molecule, then such an interaction between the surface-located polypeptide and the host may be important for the virulence of the bacterium. Compounds that interfere with the interaction of the surface-located polypeptide and the host-derived binding partner may thus be suitable for prevention or treatment of *Campylobacter jejuni* infections. Accordingly, another method of the invention relates to a method of identifying an inhibitor of the interaction of the surface-located Campylobacter jejuni polypeptide of SEQ ID NO:43 with a host-derived binding partner comprising the steps of:
a. providing the polypeptide of SEQ ID NO:43 or a fragment thereof,
b. providing a host-derived binding partner of said polypeptide (identified as described above or by any other method),
c. contacting said polypeptide with said host-derived binding partner in the absence of a putative inhibitor of said interaction,
d. contacting said polypeptide with said host-derived binding partner in the presence of said putative inhibitor, and
e. determining whether the strength of the binding of said polypeptide to said host-derived binding partner resulting from step d. is reduced as compared to that resulting from step c.

In some embodiments, step c. and d. may be performed in two different sample compartments. In other embodiments, step d. may be performed by adding the putative inhibitor to the mixture of step c. In preferred embodiments, the method is repeated for a plurality of putative inhibitors.

Of particular interest are binding partners that inhibit an activity of a surface-located polypeptide. Such activity may be enzymatic activity, transport activity, or any type of other biochemical or cellular activity, preferably enzymatic activity.
Preferred host-derived binding partners are host polypeptides and host lipids. Binding may e.g. be determined as described by Szymanski and Armstrong (1996) Infect. Immun. 64:3467-3474.
In preferred embodiments of the above described biochemical methods, the binding between the binding partner and the surface-located polypeptide or fragment thereof has a dissociation constant or Kd of less than 5 X 10⁻⁶, such as less than 10⁻⁶M, e.g. less than 5 X 10⁻⁷M, such as less than 10⁻⁷M, e.g. less than 5 X 10⁻⁸M, such as less than 10⁻⁸M, e.g. less than 5 X 10⁻⁹M, such as less than 10⁻⁹M, e.g. less than 5 X 10⁻¹⁰M, such as less than 10⁻¹⁰M, e.g. less than 5 X 10⁻¹¹M, such as less than 10⁻¹¹ M, e.g. less than 5 X 10⁻¹²M, such as less than 10⁻¹²M. Dissociation constants can e.g. be determined by surface plasmon resonance analysis.

### Cell-based methods

Reducing the level of a surface-located polypeptide, by deletion or disruption of the structural gene for it or by down-regulating gene expression (see below), may affect a bacterial cell. The cell may become more sensitive to cytotoxic compounds. Especially for surface-located polypeptides, a reduction of their level may affect the function of the cell's exterior parts, such as the outer membrane or cell wall, in preventing compounds of entering the cell. Thus, reduction of the level of an surface-located polypeptide can make a cell more 'permeable' for various compounds.

Furthermore, the invention relates to a method for identifying a compound with antibacterial activity against Campylobacter jejuni comprising the steps of
a- providing a sensitised cell which has a reduced level of the polypeptide of SEQ ID NO:43, and
b. determining the sensitivity of said cell to a putative antibacterial compound, for instance by a growth assay, wherein the putative antibacterial compound is not capable of passing through the outer membrane of a wild-type Campylobacter jejuni cell.

Preferably, the method is screening method wherein the procedure is repeated for a plurality of putative antibacterial compounds.

The rationale behind this approach is that a cell with a lower level of the surface-located polypeptide will exhibit increased sensitivity to cytotoxic compounds, allowing identification of antibacterial compounds with low potency that are missed when using wild-type cells for the assay. Compounds identified by this method will be often need to be modified in order to improve potency. This can be done by chemical modification.

Inhibition of the activity of a surface-located polypeptide may affect the viability (i.e. survival, growth and/or proliferation) of the bacterium. Of particular interest is inhibition of surface-located polypeptides that are essential for viability of Campylobacter jejuni. Essentiality of a Campylobacter jejuni gene may e.g. be investigated as described in WO 02/077183. Inhibitors of essential surface-located polypeptides may not need to enter the bacterial cell to be able to affect its viability. Thus, generally fewer requirements are posed on the structure of an inhibitor of an essential surface-located target polypeptide than on an inhibitor of an intracellular target, to be effective as an antibacterial agent.

Furthermore, the invention relates to a method for identifying an inhibitor of a polypeptide defined by of SEQ ID NO:43 comprising the steps of
a. providing two cells which differ in the level of the polypeptide of SEQ ID NO:43,
b. determining the sensitivity of said cells to a putative inhibitor, for instance by a growth assay, wherein the putative inhibitor is not capable of passing through the outermembrane of a Campylobacter jejuni cell, and
c. determining whether said two cells are differently affected by the presence of said putative inhibitor.

Preferably, the method is repeated for a plurality of putative inhibitors.

The rationale behind this approach is that the viability of a cell with a lower activity of the essential polypeptide will be more affected by an inhibitor of the polypeptide than the viability of the cell with a higher level. If the two cells are differently affected, this is an indication that the inhibitor acts on the target or at least in the same biochemical pathway.

In some embodiments of the method, the two cells with different activity of the polypeptide of interest are a wild-type cell (or other cell with wild-type activity of the gene of interest) and a sensitised cell with a reduced activity of the polypeptide of interest. In some embodiments, the different or reduced level in the sensitised cell can be a different or reduced expression level of the gene of interest (resulting in a different or reduced copy number of the polypeptide). This can be accomplished by putting the gene under control of a regulatable promoter or by regulatable expression of an antisense RNA which inhibits translation of an mRNA encoding the essential polypeptide. In other embodiments, the different or reduced activity can be a different or reduced activity of the polypeptide of interest, e.g. due to a mutation, such as a temperature-sensitive mutation.

Suitable ways of generating sensitised cells and of using these in screening for inhibitors have been described in WO 02/077183. Sensitised cells may be obtained by growing a conditional-expression *C. jejuni* mutant strain in the presence of a concentration of inducer or repressor or other conditions which provide a level of a gene product required for bacterial viability such that the presence or absence of its function becomes a rate-determining step for viability. Regulatable promoters for Campylobacter jejuni have e.g. been described in Kelana et al. (2003) J Food Prot 66:1190-1197 and Dedieu et al. (2002) Appl Environ. Microbiol. 68:4209-4215. The sub-lethal expression of the target gene may be such that growth inhibition is at least about 10%, such as at least about 25%, e.g. at least about 50%, such as at least about 75%, e.g. at least 90%, such as at least 95%.

In another embodiment of the cell-based assays of the present invention, sensitised cells are obtained by reduction of the level activity of a polypeptide required for bacterial viability using a mutation, such as a temperature-sensitive mutation, in the polypeptide. Growing such cells at an intermediate temperature between the permissive and restrictive temperatures produces cells with reduced activity of the gene product. It will be appreciated that the above method may be performed with any mutation which reduces but does not eliminate the activity or level of the gene product which is required for bacterial viability. This approach may also be combined with the conditional-expression approach. In this combined approach, cells are created in which there is a temperature-sensitive mutation in the gene of interest and in which this gene is also conditionally-expressed.

When screening for inhibitors of an essential polypeptide, growth inhibition can be measured by directly comparing the amount of growth, measured by the optical density of the culture relative to uninoculated growth medium, in an experimental sample with that of a control sample. Alternative methods for assaying cell proliferation include measuring green fluorescent protein (GFP) reporter construct emissions, various enzymatic activity assays, and other methods well known in the art. Other parameters used to measure viability include e.g. colony forming units. The above method may be performed in solid phase, liquid phase, a combination of the two preceding media, or *in vivo*. Multiple compounds may be transferred to agar plates and simultaneously tested using automated and semi-automated equipment.

Cell-based assays of the present invention are capable of detecting compounds exhibiting low or moderate potency against the target molecule of interest because such compounds are substantially more potent on sensitised cells than on non-sensitised cells. The effect may be such that a test compound may be two to several times more potent, e.g. at least 10 times more potent, such as at least 20 times more potent, e.g. at least 50 times more potent, such as at least 100 times more potent, e.g. at least 1000 times more potent, or even more than 1000 times more potent when tested on the sensitised cells as compared to non-sensitised cells.

A mutant Campylobacter jejuni strain that overexpresses a surface-located polypeptide can also be used to identify a compound that inhibits such a polypeptide. If the compound is cytotoxic, overexpression of the target polypeptide can make cells more resistant. Thus, the invention also discloses a method for finding an inhibitor of the surface-located Campylobacter jejuni polypeptide of SEQ ID NO:43 comprising the steps of
a. providing two cells which differ in the activity of the surface-located Campylobacter jejuni polypeptides of SEQ ID NO:43 wherein one cell contains a substantially wild-type copy number of said polypeptide and the other cell contains higher than wild-type copy number of said polypeptide,
b. determining the sensitivity of said cells to a putative inhibitor, for instance by a growth assay, and
c. determining whether or not said two cells are differently affected by the presence of said putative inhibitor.

Overexpression may be achieved using strong promoters or by introducing multiple copies of the structural gene for a surface-located polypeptide. Strong Campylobacter jejuni promoters have been described by Wosten et al. (1998) J. Bacteriol. 180:594-599. As also overexpression of polypeptides that are not the cellular target of an inhibitor can make cells resistance to an inhibitor, inhibition of the target polypeptide of interest by a putative inhibitor will need to be verified by other means, such as e.g. a biochemical assay.
In addition to inhibitors of a biochemical or other cellular activity of a surface-located polypeptide, the cellular methods described above can be used to identify compounds that reduce the expression level of a target, and thereby its copy number, e.g. by interfering with gene regulation.

In preferred embodiments of the any of the cell-based- or biochemical methods for finding binding partners or inhibitors, the method is repeated for a plurality of candidate compounds.

### Examples

### Strategy:

The experimental steps in the project as follows: Isolate surface proteins by low pH elution. Analyse by 2-D gels and mass spectrometry. Clone into E. coli expression vector. Produce recombinant protein, immunise mice, challenge the immunised mice with Campylobacter jejuni and look for protection against disease and intestinal colonisation. E. coli and Salmonella surface localisation is also assessed (if positive, there is potential for use in attenuated vector strains).

### Bacterial culture:

*Campylobacter jejuni* (C.j.) strain ML53 (serotype 0:19), a clinical isolate from human faeces, was donated by Karen Krogfeld (SSI). It was routinely grown on blood agar plates at 37°C in atmosphere of 10% CO₂, 5% O₂.

### Surface proteins extraction:

Bacteria were grown overnight on blood agar plates, harvested into 50 mM Tris pH7.8 and pelleted by centrifugation at 6000g for 5 minutes. The pellet was resuspended in 0.2 M glycine pH2.2 and the bacterial suspension was gently mixed at room temperature for 10 minutes. Bacteria were pelleted again by centrifugation at 6000g for 5 minutes, supernatant containing surface proteins was collected, neutralised with NaOH and frozen at -80°C. The sample was desalted on Amersham Hi-Trap desalting column before 2-D gel electrophoresis.

### 2-D gel electrophoresis:

Two-dimensional gel electrophoresis was performed either on the Ettan Dalt 2 system (Amersham Biosciences) or on the Novex NuPage system (Invitrogen) according to the manual provided with the gel system.

In brief: First dimension runs were performed on either 7 cm or 24 cm pre-cast IPG strips (pH range 3-10 or 6-11) using the Ettan IPGphor isoelectric focusing system (Amersham Biosciences) according to the manufacturer's instructions. Isofocusing was performed at the following conditions: 7 cm pH 3-10 strips:8000 Vh, 7 cm pH 6-11 strips:16000Vh, 24 cm pH 3-10 strips: 52000Vh. The second dimension was performed using pre-cast 12.5 % gels (Amersham Biosciences) at 5W per gel for 15 min then total 170 W for 4-6 hours for 24 cm strips. The 7 cm strips were run on the Novex NuPage system (Invitrogen) using pre-cast 4-12% gels (Invitrogen) at 200 volts for 40 minutes. Gels were silver stained according to a modified method described originally by Mortz et al. (2001) Proteomics 1(11), 1359-1363, and spots for mass spec analysis were picked using the Ettan Spot Picker from Amersham according to the manufacturer instructions.

### Mass Spectrometry:

Specific protein spots were spot-picked, and placed in Milli-Q water. These gel plugs were washed in 50mM NH₄HCO₃ / 50% ethanol and dehydrated by incubation in 96% ethanol. Reduction and alkylation was performed by incubating in reducing solution (10 mM DTT, 50 mM NH₄HCO₃) at 56°C followed by a room temperature incubation in alkylation solution (55 mM iodoacetamide, 50 mM NH₄HCO₃) in the dark. Two cycles of washing and dehydration were then performed prior to the addition of 5 ul trypsin solution (12.5 ng/ul Promega trypsin in 50 mM NH₄HCO₃, 10% Acetonitrile). Then an additional amount of sodium bicarbonate solution was added and the digests were incubated overnight at 37°C. Trifluoroacetic acid was added to the overnight digest followed by incubation with shaking.

Parts of the extract were used in MALDI-TOF peptide mass fingerprint analysis (Reflex IV, Bruker Daltonics, Germany) and the peaklist was used in database searching against a specific Campylobacter jejuni database. The Mascot search program and scoring algorithm (Matrix Science, UK) was used in database searching. Peptide mass tolerance was set to 60 ppm and 0.5 Da, respectively. Search parameters were adjusted to include oxidation of Met, the addition of Carbamidomethyl groups to Cys, and trypsin was allowed to miss one cleavage site per peptide.
In total 51 different Campylobacter jejuni proteins were identified using this procedure. The full-length sequences of these proteins are given in SEQ ID NO:1-51 (see figure 4). The proteins in the sequence listing are functionally classified according to the classification from the Sanger Institute. All proteins which were predicted from genomic sequence but had not been characterised before, and did not have any homology to previously characterised proteins, were classified as hypothetical by the Sanger institute. Those which had homology to known proteins were named as the corresponding known protein with the pre-fix "probable" or "putative", depending on the degree of homology. Some proteins have small motifs, which did not allow to assign a precise biochemical/metabolic function to them, but did allow to state the fact that they possess of a certain feature, such as nucleotide binding motifs, membrane attachment sites, etc. Putative periplasmic proteins were classified as such on a similar basis - they possess a short (4-6 amino acids) N-terminal motif, which may mediate transport to the periplasm.

### Bioinformatic studies:

Antigenicity index studies were performed using the default parameters determined using Lasergene sequence analysis software from the company DNAStar (Burland, TG (2000) Methods Mol. Biol. 132:71-91). The sequences set forth in SEQ ID NO:52-119 are predicted to be particularly antigenic fragments of their corresponding full-length polypeptides (see figure 4).

### Cloning and expression in E. coli:

Genes corresponding to the proteins of interest were PCR amplified and cloned into an E. coli expression vector containing his-tag (pET101, Invitrogen). Resulting plasmids were transformed into E. coli BL21 (DE3) strain and protein expression was induced with 0.5 mM IPTG for 4 hours. Glycine eluate was prepared as above and analysed for the presence of the recombinant Campylobacter jejuni protein by three independent methods: Coomassie staining, Western blotting with anti-his tag antibody, and mass spec analysis. Surface localisation detection was carried out for eight plasmids (the ones expressing Cj0092, Cj0143c, Cj0420, Cj0715, Cj0772c, Cj1018c, Cj1380 and Cj1643) All eight proteins were found on the cell-surface of E. coli.

Recombinant protein was purified from cultures grown and induced as described above using NTA-Ni agarose (Qiagen) according to the manufacturer's instructions. Purified proteins were dialyzed against PBS and kept frozen at -80.

### Mouse immunization

### Antigen dose determination.

To determine optimal immunization doze and vaccination time schedule for each protein, mice were immunized with 1, 5, 10 and 25 microgram of each protein. Appropriate amount of protein dissolved in PBS was mixed with 100 µg alum (Alhydrogel, Brenntag Biosector) /mice and rotated at room temperature for 30 minutes. Volume was adjusted to 0.5 ml with PBS. Mice were immunized subcutaneously 3 times (day 0, 14, 28). Bleeds were taken on day 0 and 7 days after each immunization (day 7, 21, 35). A blood sample of 200 µl blood ml was drawn from the retro orbital plexus at day 7, left for 1 hour to coagulate, centrifuged for 10 min at 3500g, and serum was collected.

### Bleed analysis

Antibody response was monitored by determining the antibody titer in the bleeds. This was done by Western blots. Serum was serially diluted in PBS/0.1% Tween from 1:100 to 1:25600. Recombinant protein was run on preparative (2-D) well gels (Invitrogen) so that 1.5 mm of the protein band on the membrane would correspond to 100 ng of the protein. Surf-Blot apparatus with 1.5 mm wide slots (idea Scientific, Minneapolis, MN) was used to perform Western blotting. Serum dilutions were used as primary antibody and alkaline phosphatase conjugated anti-mouse antibodies (Sigma) were used as secondary antibody. Blots were developed with FastTab tablets (Sigma). All antigens were detectable at an antiserum dilution between 12800 and 25600 when applied at 5, 10 and 25 microgram/animal.

### Immunization for challenge

Mice were immunized with 25 microgram of recombinant protein vaccine prepared as above, on days 0,14 and 28. Bleeds were taken on days 7, 21 and 35 and tested as above. Mice, which were not used for challenge experiments within two weeks from the last boost, were boosted again with 10 microgram of recombinant protein one week before the challenge.

### Mouse oral challenge

*Campylobacter jejuni* strains ML1 and ML53 (clinical isolates donated by Karen Krogfeld, SSI) were grown o/n on blood agar plates at 42°. The day before challenge, bacteria were harvested into Brain heard infusion broth containing 1% yeast extract and diluted to OD₆₀₀ 0.05 to obtain a starter culture. 75cm² flasks containing 20ml of BHI agar were seeded with 25ml of the starter culture and incubated overnight at 42°. On the day of challenge, bacteria were harvested by centrifugation at 6000g for 5 minutes and resuspended in fresh BHI/YE broth to OD₆₀₀ 1 (5x10⁸ to 10⁹ colony forming units (cfu)/ml; actual cfu was determined for each batch by plating serial dilutions on BHI agar plates). Suspension was kept on ice until it given to mice (usually within 3 hours of preparation).

6-8 vaccinated mice per antigen were challenged orally with 2.5-5x10⁸cfu of *Campylobacter jejuni* (0.5ml of the suspension prepared as above). Five faecal pellets (or all faecal pellets in ACE017 experiment) were collected each day, placed into 0.5ml (or 5ml) of BHI/YE broth supplemented with 10% glycerol and frozen at -80°.

Colonization was monitored by plating on selective media for Campylobacter. Faecal pellets were homogenized, volume adjusted to 2 (or 12) ml, and 250ul of the homogenate was placed into 1.5 ml of BHI/YE broth on top of the blood agar plate. Plates were incubated at 42° for 2-3 hours, and 150-250ul of the liquid was spread on Karmali agar plates, which were then incubated at 42° for 48 hours. Plates were scored as follows: a score of 1000 was assigned if confluent lawn was observed, a score of 500 if separate colonies were distinguishable in the lawn, and a score of 0-100, if the colony number was below 100 (colonies were counted in that case). Score was averaged across all mice for every antigen on every day.

### Strain screening

13 strains of Campylobacter jejuni and one strain of Campylobacter coli were screened for the presence of the 8 antigens used in mouse challenge experiments. Whole cell lysed was run on SDS gel and Western blotted with antisera from immunized mice.

### Results:

Five experiments including different combinations of antigens and controls were performed as listed below:

| Experiment name | Antigens used |
|---|---|
| ACE003b | Cj0092, Cj0143c, Cj0420, Cj0772c, alum |
| ACE003c | Cj0715, Cj1018c, Cj1380, Cj1643, alum |
| ACE011 a | Cj0092, Cj0143c, Cj0420, Cj0772c, alum |
| ACE011 b | Cj0715, Cj1018c, Cj1380, Cj1643, alum |
| ACE017 | Cj0092, Cj0143c, Cj0420, Cj0772c, Cj1018c, Cj1380, Cj1643, cellulose-binding domain from Clostridium cellulovorans (CBP, Sigma Aldrich C8581), glycine eluate from C.j. strains ML1 and ML53. |

Example of bleed analysis performed on bleeds from mice from experiments ACE003b and ACE003c are presented on figures 1 and 2.

Results of challenge are presented in Figure 3.

Results of screening for the presence of antigens in different Campylobacter strains are presented in table 1.

### DNA sequencing of ACE 393 from the ML53 (serotype 0:19) strain:

The DNA sequencing of ACE 393 (SEQ ID NO:120) from strain ML53 revealed 3 differences compared to the published sequence at NCBI on the amino acid level after translating into the protein sequence.

The differences were the following:
Position 70 of the amino acid sequence: V (public sequence) to A (ML53 strain)
Position 136 of the amino acid sequence: A (public sequence) to T (ML53 strain)
Position 168 of the amino acid sequence: T (public sequence) to A (ML53 strain)

An experimental verification of the protein sequence of recombinant ACE 393 from the strain ML53 (0:19) that was used for the initial animal efficacy studies was carried out. The analysis included: Intact mass determination by MALDI-TOF; 1 D Gel analysis; 2D Gel analysis and 2D Gel western blot. The data (not shown) was based on the initial construct, that is, the sequence including a C-terminal 6 His-tag and a V5-linker region. All assays and analyses were conducted with ACE 393 obtained after Ni-His purification according to standard protocols.

From the intact analysis of ACE 393 two proteins were seen. These two masses matched perfectly the predicted mass of an N-terminal truncated form (- 21 amino acids and a similar N-terminal truncated form with additional C-terminal truncation of 19 amino acids. 1 D SDS PAGE showed two spots one major and one minor. This corresponded well with the data from the intact MALDI-TOF analysis. The peptide mapping data from these two spots did not show any difference possibly due to overloading. The numbers of spots on 2D gel analysis indicated that the mixture might contain more than two components. The data indicated that the changes might be due to differences in charge due to the number of SDS molecules. Western blot data using the mouse antibodies gave the same pattern and fitted well with the silver stain data.

### MALDI-TOF peptide fingerprint analysis of native and recombinant ACE 393

### Native-form

The native form of ACE 393 was found in 4 different 2D gels containing whole Campylobacter cell lysate. The identification was based on MALDI-TOF peptide fingerprint analysis with a sequence-coverage of 40 to 55%.

The MALDI-TOF peptide fingerprint data was searched against both the public sequence and the ACE-393 in-house found sequence (ML53, 0:19). The in-house sequence gave two additional peptides:

| | |
|---|---|
| 131-143 | DIVLDTEIGGVAK (SEQ ID NO:121) |
| 166-190 | FAASTSTITLSDDINLNIEVEANEK (SEQ ID NO:122) |

| | |
|---|---|
| (Amino acids in bold are the differences between the two sequences) | |

The peptide containing the last modification compared to the public sequence (68-73; LDATIK (SEQ ID NO:123)) is too small to be found by MALDI-TOF peptide fingerprint analysis.

### Recombinant form

MALDI-TOF peptide fingerprint analysis of the recombinant protein revealed the same two modifications as found in the analysis of the native protein. Additional peptides were identified from the linker-His sequence.

### Hypervariable regions

Target sequences were checked against potential hypervariable and homopolymeric regions described in "The genome sequence of the food-borne pathogen Campylobacter jejuni reveals hypervariable sequences. Nature. 2000 Feb 10;403(6770):665-8." The targets described herein were not found in these variable regions, thus making them particularly suitable as vaccine candidates.

## Claims

1. A composition comprising
- a polypeptide which comprises the sequence of SEQ ID NO:43 wherein X₁ is V or A and X₂ is A or T and X₃ is T or A, or comprises an antigenic fragment selected from the group consisting of SEQ ID NO: 74-81 or a variant of said sequence having at least 75% sequence identity to said sequence,
- a polynucleotide comprising a sequence encoding said polypeptide,
- an expression vector comprising a sequence encoding said polypeptide,
- a recombinant virus or recombinant cell comprising said polynucleotide or said expression vector, or
- an antibody capable of binding said polypeptide,
for use as a medicament.

2. The composition of claim 1, wherein the composition comprises
- a polypeptide which comprises the sequence of SEQ ID NO:43 wherein X₁ is V or A and X₂ is A or T and X₃ is T or A, or comprises an antigenic fragment selected from the group consisting of SEQ ID NO: 74-81 or a variant of said sequence having at least 75% sequence identity to said sequence,
- a polynucleotide comprising a sequence encoding said polypeptide,
- an expression vector comprising a sequence encoding said polypeptide, or
- a recombinant virus or recombinant cell comprising said polynucleotide or said expression vector.

3. The composition of any of the preceding claims, wherein said polynucleotide comprises the sequence of SEQ ID NO:120.

4. The composition of any of the preceding claims, wherein the variant has at least 95%, such as at least 96%, e.g. at least 97%, such as at least 98%, e.g. at least 99% sequence identity to said sequence.

5. The composition of any of the preceding claims, wherein the polypeptide comprises a tag, such as a histidine tag.

6. The composition of any of the preceding claims, wherein the recombinant cell is an attenuated or reduced-virulence Escherichia coli cell or an attenuated or reduced-virulence Salmonella cell.

7. The composition of any of the preceding claims, wherein the recombinant cell is alive.

8. The composition of any of the preceding claims, wherein the recombinant cell is dead.

9. The composition of any of claims 2-8, wherein the medicament is a vaccine.

10. The composition of claim 9, wherein the composition comprises an immunogenic carrier, such as a carrier protein, wherein the immunogenic carrier preferably is bound to said polypeptide.

11. The composition of any of claims 9-10, wherein the composition comprises an adjuvant.

12. The composition of claim 1, wherein the composition comprises an antibody capable of binding the polypeptide of SEQ ID NO: 43 wherein X₁ is V or A and X₂ is A or T and X₃ is T or A.

13. The composition of claim 12, wherein the antibody furthermore is capable of binding an intact Campylobacter jejuni cell.

14. The composition of any of claims 12 to 13, wherein the antibody is polyclonal.

15. The composition of any of claims 12 to 13, wherein the antibody is monoclonal.

16. The composition of any of claims 12 to 15, wherein the antibody is a human antibody or humanised antibody.

17. The composition of any of claims 12 to 13, wherein the antibody is a binding fragment of an antibody selected from the group consisting of Fab, Fab', F(ab)₂, and Fv.

18. The composition of any of the preceding claims, wherein the composition comprises a pharmaceutically-acceptable carrier.

19. The composition of any of the preceding claims, wherein the composition is suitable for systemic administration.

20. The composition of any of the preceding claims, wherein the composition is suitable for intravenous, intramuscular, or subcutaneous administration.

21. The composition of any of the preceding claims, wherein the composition is suitable for oral administration.

22. The composition of any of the preceding claims, wherein the composition is suitable for intranasal administration.

23. An antibody capable of binding the polypeptide of SEQ ID NO:43 wherein X₁ is V or A and X₂ is A or T and X₃ is T or A or an antigenic fragment selected from the group consisting of SEQ ID NO: 74-81 or a variant of said sequence having at least 75% sequence identity to said sequence, for use as a medicament.

24. The antibody of claim 23, wherein the antibody furthermore is capable of binding an intact Campylobacter jejuni cell.

25. The antibody of any of claims 23 to 24, wherein the antibody is polyclonal.

26. The antibody of any of claims 23 to 24, wherein the antibody is monoclonal.

27. The antibody of any of claims 23 to 26, wherein the antibody is a human antibody or humanised antibody.

28. The antibody of any of claims 23 to 24, wherein the antibody is a binding fragment of an antibody.

29. A recombinant attenuated or reduced-virulence Escherichia coli or recombinant attenuated or reduced-virulence Salmonella cell transformed or transfected with a polynucleotide comprising a sequence encoding the polypeptide of SEQ ID NO:43 wherein X₁ is V or A and X₂ is A or T and X₃ is T or A, or encoding an antigenic fragment selected from the group consisting of SEQ ID NO: 74-81 or a variant of said sequence having at least 75% sequence identity to said sequence, for use as a medicament.

30. Use of
- a polypeptide which comprises the sequence of SEQ ID NO:43 wherein X₁ is V or A and X₂ is A or T and X₃ is T or A, or comprises an antigenic fragment selected from the group consisting of SEQ ID NO: 74-81 or a variant of said sequence having at least 75% sequence identity to said sequence
- a polynucleotide comprising a sequence encoding said polypeptide,
- an expression vector comprising a sequence encoding said polypeptide, or
- a recombinant virus or recombinant cell comprising said polynucleotide or said expression vector,
for the preparation of a medicament for the immunisation of an animal or human being against Campylobacter, preferably Campylobacter jejuni, infections.

31. The use of claim 30, wherein the immunisation induces a protective immune response.

32. The use of claim 30 or 31, wherein the medicament is a medicament suitable for parenteral, intravenous, intramuscular, subcutaneous, oral or intranasal administration.

33. The use of any of claims 30-32, wherein the medicament further comprises an adjuvant.

34. Use of an antibody capable of binding the polypeptide of SEQ ID NO:43 wherein X₁ is V or A and X₂ is A or T and X₃ is T or A or an antigenic fragment selected from the group consisting of SEQ ID NO: 74-81 or a variant of said sequence having at least 75% sequence identity to said sequence, preferably an antibody as defined in any of claims 23 to 28, for the manufacture of a medicament for the treatment or prevention of Campylobacter, preferably Campylobacter jejuni, infections in an animal or human being.

35. A method for detecting Campylobacter jejuni in a sample comprising the steps of
a. contacting said sample with an indicator moiety capable of specifically binding the polypeptide of SEQ ID NO:43 wherein X₁ is V or A and X₂ is A or T and X₃ is T or A, wherein the indicator moiety furthermore is capable of specifically binding intact Campylobacter jejuni cells, and
b. determining whether a signal has been generated by the indicator moiety, thereby detecting whether said sample contains Campylobacter jejuni.

36. The method of claim 35, wherein said indicator moiety does not pass through the outer membrane of a Campylobacter jejuni cell.

37. The method of any of claims 35 to 36, wherein said indicator moiety consist of or comprises an antibody, such as an antibody as defined in any of claims 12 to 17.

38. A method for identifying an inhibitor of the polypeptide of SEQ ID NO:43 wherein X₁ is V or A and X₂ is A or T and X₃ is T or A, comprising the steps of
a. providing two cells which differ in the level of the polypeptide of SEQ ID NO:43 wherein X₁ is V or A and X₂ is A or T and X₃ is T or A,
b. determining the sensitivity of said cells to a putative inhibitor, for instance by a growth assay, wherein the putative inhibitor is not capable of passing through the outer membrane of a Campylobacter jejuni cell, and
c. determining whether said two cells are differently affected by the presence of said putative inhibitor.

39. A method for identifying a host-derived binding partner of the polypeptide of SEQ ID NO:43 wherein X₁ is V or A and X₂ is A or T and X₃ is T or A or an antigenic fragment selected from the group consisting of SEQ ID NO: 74-81, comprising the steps of
a. providing purified and electrophoretically separated host membranes blotted over to a membrane,
b. incubating said host membranes with the polypeptide of SEQ ID NO:43 wherein X₁ is V or A and X₂ is A or T and X₃ is T or A or an antigenic fragment selected from the group consisting of SEQ ID NO: 74-81,
c. detecting binding by using antibodies specific for the polypeptide of SEQ ID NO:43 wherein X₁ is V or A and X₂ is A or T and X₃ is T or A or an antigenic fragment selected from the group consisting of SEQ ID NO: 74-81, and
d. identifying the binding partner by elution from said blot membrane and subsequent analysis.

## Patentansprüche

1. Zusammensetzung umfassend,
- ein Polypeptid, das die Sequenz von SEQ ID Nr. 43 umfasst, worin X₁ V oder A und X₂ A oder T und X₃ T oder A ist, oder ein antigenes Fragment umfasst, ausgewählt aus der Gruppe bestehend aus SEQ ID Nr. 74-81 oder eine Variante der Sequenz mit mindestens 75 % Sequenzidentität zu der Sequenz,
- ein Polynukleotid umfassend eine Sequenz, die das Polypeptid kodiert,
- einen Expressionsvektor, der eine Sequenz umfasst, die das Polypeptid kodiert,
- ein rekombinantes Virus oder eine rekombinante Zelle, die das Polynukleotid oder den Expressionsvektor umfasst, oder
- einen Antikörper, der das Polypeptid binden kann,
zur Verwendung als Medikament.

2. Zusammensetzung nach Anspruch 1, worin die Zusammensetzung umfasst,
- ein Polypeptid, das die Sequenz von SEQ ID Nr. 43 umfasst, worin X₁ V oder A und X₂ A oder T und X₃ T oder A ist, oder ein antigenes Fragment umfasst, ausgewählt aus der Gruppe bestehend aus SEQ ID Nr. 74-81 oder eine Variante der Sequenz mit mindestens 75 % Sequenzidentität zu der Sequenz,
- ein Polynukleotid umfassend eine Sequenz, die das Polypeptid kodiert,
- einen Expressionsvektor, der eine Sequenz umfasst, die das Polypeptid kodiert, oder
- ein rekombinantes Virus oder eine rekombinante Zelle, die das Polynukleotid oder den Expressionsvektor umfasst.

3. Zusammensetzung nach einem der vorstehenden Ansprüche, worin das Polynukleotid die Sequenz von SEQ ID Nr. 120 umfasst.

4. Zusammensetzung nach einem der vorstehenden Ansprüche, worin die Variante mindestens 95 %, beispielsweise mindestens 96 %, beispielweise mindestens 97 %, beispielsweise mindestens 98 %, beispielsweise mindestens 99 % Sequenzidentität zu der Sequenz aufweist.

5. Zusammensetzung nach einem der vorstehenden Anspruchs, worin das Polypeptid einen Tag, beispielsweise ein Histidin-Tag umfasst.

6. Zusammensetzung nach einem der vorstehenden Ansprüchen, worin die rekombinante Zelle eine attenuierte oder vermindert virulente *Escherichia coli* Zelle oder eine attenuierte oder vermindert virulente Salmonella Zelle ist.

7. Zusammensetzung nach einem der vorstehenden Ansprüche, worin die rekombinante Zelle lebend ist.

8. Zusammensetzung nach einem der Anspruche 1-6, worin die rekombinante Zelle tot ist.

9. Zusammensetzung nach einem der Ansprüche 2-8, worin das Medikament ein Impfstoff ist.

10. Zusammensetzung nach Anspruch 9, worin die Zusammensetzung einen immunogenen Träger, beispielsweise ein Trägerprotein umfasst, worin der immunogene Träger vorzugsweise an das Polypeptid gebunden ist.

11. Zusammensetzung nach einem der Ansprüche 9-10, worin die Zusammensetzung ein Adjuvanz umfasst.

12. Zusammensetzung nach Anspruch 1, worin die Zusammensetzung einen Antikörper umfasst, der das Polypeptid von SEQ ID Nr. 43 binden kann, worin X₁ V oder A und X₂ A oder T und X₃ T oder A ist.

13. Zusammensetzung nach Anspruch 12, worin der Antikörper weiterhin an eine intakte *Campylobacter jejuni* Zelle binden kann.

14. Zusammensetzung nach einem der Ansprüche 12 bis 13, worin der Antikörper polyklonal ist.

15. Zusammensetzung nach einem der Ansprüche 12 bis 13, worin der Antikörper monoklonal ist.

16. Zusammensetzung nach einem der Ansprüche 12 bis 15, worin der Antikörper ein menschlicher Antikörper oder ein humanisierter Antikörper ist.

17. Zusammensetzung nach einem der Ansprüche 12 bis 13, worin der Antikörper ein bindendes Fragment eines Antikörpers ist, ausgewählt aus der Gruppe bestehend aus Fab, Fab', F(ab)₂, und Fv.

18. Zusammensetzung nach einem der vorstehenden Ansprüche, worin die Zusammensetzung einen pharmazeutisch verträglichen Träger umfasst.

19. Zusammensetzung nach einem der vorstehenden Ansprüche, worin die Zusammensetzung für eine systemische Verabreichung geeignet ist.

20. Zusammensetzung nach einem der vorstehenden Ansprüche, worin die Zusammensetzung für eine intravenöse, intramuskuläre oder subkutane Verabreichung geeignet ist.

21. Zusammensetzung nach einem der vorstehenden Ansprüche, worin die Zusammensetzung für eine orale Verabreichung geeignet ist.

22. Zusammensetzung nach einem der vorstehenden Ansprüche, worin die Zusammensetzung für eine intranasale Verabreichung geeignet ist.

23. Antikörper, der das Polypeptid von SEQ ID Nr. 43, worin X₁ V oder A und X₂ A oder T und X₃ T oder A ist, oder ein antigenes Fragment binden kann, ausgewählt aus der Gruppe bestehend aus SEQ ID Nr. 74-81 oder eine Variante der Sequenz mit mindestens 75 % Sequenzidentität zu der Sequenz, zur Verwendung als Medikament.

24. Antikörper nach Anspruch 23, worin der Antikörper weiterhin eine intakte *Campylobacter jejuni* Zelle binden kann.

25. Antikörper nach einem der Ansprüche 23 bis 24, worin der Antikörper polyklonal ist.

26. Antikörper nach einem der Ansprüche 23 bis 24, worin der Antikörper monoklonal ist.

27. Antikörper nach einen der Ansprüche 23 bis 26, worin der Antikörper ein menschlicher Antikörper oder ein humanisierter Antikörper ist.

28. Antikörper nach einem der Ansprüche 23 bis 24, worin der Antikörper ein bindendes Fragment eines Antikörpers ist.

29. Rekombinante attenuierte oder vermindert virulente *Escherichia coli* oder rekombinante attenuierte oder vermindert virulente Salmonella Zelle, die mit einem Polynukleotid transformiert oder transfiziert ist, das eine Sequenz umfasst, die das Polypeptid von SEQ ID Nr. 43 kodiert, worin X₁ V oder A und X₂ A oder T und X₃ T oder A ist, oder ein antigenes Fragment kodiert, ausgewählt aus der Gruppe bestehend aus SEQ ID Nr. 74-81 oder eine Variante der Sequenz, die mindestens 75 % Sequenzidentität zu der Sequenz aufweist, zur Verwendung als Medikament.

30. Verwendung
- eines Polypeptids, das die Sequenz von SEQ ID Nr. 43 umfasst, worin X₁ V oder A und X₂ A oder T und X₃ T oder A ist, oder ein antigenes Fragment umfasst, ausgewählt aus der Gruppe bestehend aus SEQ ID Nr. 74-81 oder eine Variante der Sequenz mit mindestens 75 % Sequenzidentität zu der Sequenz,
- ein Polynukleotid umfassend eine Sequenz, die das Polypeptid kodiert,
- einen Expressionsvektor, der eine Sequenz umfasst, die das Polypeptid kodiert, oder
- ein rekombinantes Virus oder eine rekombinante Zelle, die das Polynukleotid oder den Expressionsvektor umfasst,
für die Herstellung eines Medikaments für die Immunisierung eines Tieres oder Menschen gegen Infektionen durch Campylobacter, vorzugsweise *Campylobacter jejuni.*

31. Verwendung nach Anspruch 30, wobei die Immunisierung eine schützende Immunantwort hervorruft.

32. Verwendung nach einem der Ansprüche 30 oder 31, wobei das Medikament ein Medikament ist, das für eine parenterale, intravenöse, intramuskuläre, subkutane, orale oder intranasale Verabreichung geeignet ist.

33. Verwendung nach einem der Ansprüche 30-32, wobei das Medikament weiterhin ein Adjuvanz umfasst,

34. Verwendung eines Antikörpers, der das Polypeptid von SEQ ID Nr. 43, worin X₁ V oder A und X₂ A oder T und X₃ T oder A ist, oder ein antigenes Fragment binden kann, ausgewählt aus der Gruppe bestehend aus SEQ ID Nr. 74-81 oder eine Variante der Sequenz mit mindestens 75 % Sequenzidentität zu der Sequenz, vorzugsweise einen Antikörper, der wie in den Ansprüchen 23 bis 28 definiert ist, für die Herstellung eines Medikaments für die Behandlung oder Vorbeugung von Infektionen mit Campylobacter, vorzugsweise *Campylobacter jejuni* in einem Tier oder einem Menschen.

35. Verfahren zum Nachweisen von *Campylobacter jejuni* in einer Probe, umfassend die Schritte,
a. Inkontaktbringen der Probe mit einem Indikatorrest, der das Polypeptid von SEQ ID Nr. 43 spezifisch binden kann, worin X₁ V oder A und X₂ A oder T und X₃ T oder A ist, worin der Indikatorrest weiterhin intakte *Camplylobacter jejuni* Zellen spezifisch binden kann, und
b. Bestimmen, ob durch den Indikatorrest ein Signal erzeugt wurde, wodurch nachgewiesen wird, ob die Probe *Campylobacter jejuni* enthält.

36. Verfahren nach Anspruch 35, wobei der Indikatorrest nicht durch die äußere Membran einer *Campylobacter jejuni* Zelle passt.

37. Verfahren nach einem der Ansprüche 35 bis 36, wobei der Indikatorrest aus einem Antikörper besteht oder einen umfasst, beispielsweise einen Antikörper, der in einem der Ansprüche 12 bis 1 definiert ist.

38. Verfahren zum Identifizieren eines Hemmstoffs des Polypeptids von SEQ ID Nr.43, worin X₁ V oder A und X₂ A oder T und X₃ T oder A ist, umfassend die Schritte,
a. Bereitstellen von zwei Zellen, die sich in dem Niveau des Polypeptids von SEQ ID Nr. 43, worin X₁ V oder A und X₂ A oder T und X₃ T oder A ist, unterscheiden,
b. Bestimmen der Empfindlichkeit der Zellen auf den mutmaßlichen Hemmstoff, beispielsweise durch einen Wachstumstest, wobei der mutmaßliche Hemmstoff nicht durch die äußere Membran einer *Campylobacter jejuni* Zelle passt, und
c. Bestimmen, ob die zwei Zellen durch die Anwesenheit des mutmaßlichen Hemmstoffs unterschiedlich beeinflusst werden.

39. Verfahren zum Identifizieren eines von einem Wirt stammenden Bindungspartners des Polypeptids von SEQ ID Nr. 43, worin X₁ V oder A und X₂ A oder T und X₃ T oder A ist oder eines antigenen Fragments, ausgewählt aus der Gruppe bestehend aus SEQ ID Nr. 74-81, umfassend die Schritte,
a. Bereitstellen von aufgereinigten und elektrophoretisch getrennten auf eine Membran übertragene Wirts Membranen,
b. Inkubieren der Wirts-Membranen mit dem Polypeptid von SEQ ID Nr. 43, worin X₁ V oder A und X₂ A oder T und X₃ T oder A ist, oder einem antigenen Fragment, ausgewählt aus der Gruppe bestehend aus SEQ ID Nr. 74-81,
c. Nachweisen einer Bindung unter Verwendung von für das Polypeptid von SEQ ID Nr. 43 spezifischen Antikörpern, worin X₁ V oder A und X₂ A oder T und X₃ T oder A ist, oder einem antigenen Fragment, ausgewählt aus der Gruppe bestehend aus SEQ ID Nr. 74-81, und
d. Identifizieren des Bindungspartners durch Elution von der Blot-Membran und anschließender Analyse.

## Revendications

1. Composition comprenant
- un polypeptide qui comprend la séquence de SEQ ID NO : 43 dans laquelle X₁ représente V ou A et X₂ représente A ou T et X₃ représente T ou A, ou comprend un fragment antigénique choisi dans le groupe comprenant SEQ ID NO : 74 à 81 ou un variant de ladite séquence présentant au moins 75 % d'identité de séquence avec ladite séquence,
- un polynucléotide comprenant une séquence codant pour ledit polypeptide,
- un vecteur d'expression comprenant une séquence codant pour ledit peptide,
- un virus recombinant ou une cellule recombinante comprenant ledit polynucléotide ou ledit vecteur d'expression, ou
- un anticorps capable de lier ledit polypeptide,
pour une utilisation en tant que médicament.

2. Composition selon la revendication 1, où la composition comprend
- un polypeptide qui comprend la séquence de SEQ ID NO : 43 dans laquelle X₁ représente V ou A et X₂ représente A ou T et X₃ représente T ou A, ou comprend un fragment antigénique choisi dans le groupe comprenant SEQ ID NO : 74 à 81 ou un variant de ladite séquence présentant au moins 75 % d'identité de séquence avec ladite séquence,
- un polynucléotide comprenant une séquence codant pour ledit polypeptide,
- un vecteur d'expression comprenant une séquence codant pour ledit peptide, ou
- un virus recombinant ou une cellule recombinante comprenant ledit polynucléotide ou ledit vecteur d'expression.

3. Composition selon l'une quelconque des revendications précédentes, dans laquelle ledit polynucléotide comprend la séquence de SEQ ID NO : 120.

4. Composition selon l'une quelconque des revendications précédentes, dans laquelle le variant présente au moins 95 %, comme au moins 96 %, par exemple au moins 97 %, comme au moins 98 %, par exemple au moins 99 % d'identité de séquence avec ladite séquence.

5. Composition selon l'une quelconque des revendications précédents, dans laquelle le polypeptide comprend un marqueur, comme un marqueur histidine.

6. Composition selon l'une quelconque des revendications précédentes, dans laquelle la cellule recombinante est une cellule de *Escherichia coli* de virulence atténuée ou réduite ou une cellule de *Salmonella* de virulence atténuée ou réduite.

7. Composition selon l'une quelconque des revendications précédentes, dans laquelle la cellule recombinante est vivante.

8. Composition selon l'une quelconque des revendications précédentes, dans laquelle la cellule recombinante est morte.

9. Composition selon l'une quelconque des revendications 2 à 8, où le médicament est un vaccin.

10. Composition selon la revendication 9, où la composition comprend un support immunogène, tel qu'une protéine de transport, où le support immunogène est de préférence lié au dit polypeptide.

11. Composition selon l'une quelconque des revendications 9 ou 10, où la composition comprend un adjuvant.

12. Composition selon la revendication 1, où la composition comprend un anticorps capable de lier le polypeptide de SEQ ID NO : 43 dans laquelle X₁ représente V ou A et X₂ représente A ou T et X₃ représente T ou A.

13. Composition salon la revendication 12, dans laquelle l'anticorps est en outre capable de lier une cellule intacte de *Campylobacter jejuni*.

14. Composition selon l'une quelconque des revendications 12 ou 13, dans laquelle l'anticorps est polyclonal.

15. Composition selon l'une quelconque des revendications 12 ou 13, dans laquelle l'anticorps est monoclonal.

16. Composition selon l'une quelconque des revendications 12 à 15, dans laquelle l'anticorps est un anticorps humain ou un anticorps humanisé.

17. Composition selon l'une quelconque des revendications 12 ou 13, dans laquelle l'anticorps est un fragment de liaison d'un anticorps choisi dans le groupe comprenant Fab, Fab', F(ab)₂ et Fv.

18. Composition selon l'une quelconque des revendications précédentes, où la composition comprend un support pharmaceutiquement acceptable.

19. Composition selon l'une quelconque des revendications précédentes, où la composition est appropriée pour une administration systémique.

20. Composition selon l'une quelconque des revendications précédentes, où la composition est appropriée pour une administration intraveineuse, intramusculaire ou sous-cutanée.

21. Composition selon l'une quelconque des revendications précédentes, où la composition est appropriée pour une administration orale.

22. Composition selon l'une quelconque des revendications précédentes, où la composition est appropriée pour une administration intranasale.

23. Anticorps capable de lier le polypeptide de SEQ ID NO : 43 dans laquelle X₁ représente V ou A et X₂ représente A ou T et X₃ représente T ou A ou un fragment antigénique choisi dans le groupe comprenant SEQ ID NO : 74 à 81 ou un variant de ladite séquence présentant au moins 75 % d'identité de séquence avec ladite séquence, pour une utilisation en tant que médicament.

24. Anticorps selon la revendication 23, où l'anticorps est en outre capable de lier une cellule intacte de *Campylobacter jejuni.*

25. Anticorps selon l'une quelconque des revendications 23 ou 24, où l'anticorps est polyclonal.

26. Anticorps selon l'une quelconque des revendications 23 ou 24, où l'anticorps est monoclonal.

27. Anticorps selon l'une quelconque des revendications 23 à 26, où l'anticorps est un anticorps humain ou un anticorps humanisé.

28. Anticorps selon l'une quelconque des revendications 23 ou 24, où l'anticorps est un fragment de liaison d'un anticorps.

29. Cellule recombinant de *Escherichia coli* de virulence atténuée ou réduite ou cellule recombinante de *Salmonella* de virulence atténuée ou réduite transformée ou transfectée avec un polynucléotide comprenant une séquence codant pour le polypeptide de SEQ ID NO : 43 dans laquelle X₁ représente V ou A et X₂ représente A ou T et X₃ représente T ou A, ou codant pour un fragment antigénique choisi dans le groupe comprenant SEQ ID NO : 74 à 81 ou un variant de ladite séquence présentant au moins 75 % d'identité de séquence avec ladite séquence, pour une utilisation en tant que médicament.

30. Utilisation
- d'un polypeptide qui comprend la séquence de SEQ ID NO : 43 dans laquelle X₁ représente V ou A et X₂ représente A ou T et X₃ représente T ou A, ou comprend un fragment antigénique choisi dans le groupe comprenant SEQ ID NO : 74 à 81 ou un variant de ladite séquence présentant au moins 75 % d'identité de séquence avec ladite séquence,
- d'un polynucléotide comprenant une séquence codant pour ledit polypeptide,
- d'un vecteur d'expression comprenant une séquence codant pour ledit peptide, ou
- d'un virus recombinant ou d'une cellule recombinante comprenant ledit polynucléotide ou ledit vecteur d'expression,
pour la fabrication d'un médicament destiné à l'immunisation d'un animal ou d'un être humain contre des infections à *Campylobacter,* de préférence *Campylobacter jejuni.*

31. Utilisation selon la revendication 30, où l'immunisation induit une réponse immunitaire protectrice.

32. Utilisation selon la revendication 30 ou 31, où le médicament est un médicament approprié pour une administration parentérale, intraveineuse, intramusculaire, sous-cutanée, orale ou intranasale.

33. Utilisation selon l'une quelconque des revendications 30 à 32, où le médicament comprend en outre un adjuvant.

34. Utilisation d'un anticorps capable de lier le polypeptide de SEQ ID NO : 43 dans laquelle X₁ représente V ou A et X₂ représente A ou T et X₃ représente T ou A, ou un fragment antigénique choisi dans le groupe comprenant SEQ ID NQ : 74 à 81 ou un variant de ladite séquence présentant au moins 75 % d'identité de séquence avec ladite séquence, de préférence un anticorps tel que défini dans l'une quelconque des revendications 23 à 28, pour la fabrication d'un médicament destiné au traitement ou à la prévention d'infections à *Campylobacter,* de préférence *Campylobacter jejuni,* chez un animal ou un être humain.

35. Procédé de détection de *Campylobacter jejuni* dans un échantillon comprenant les étapes consistant à
a. mettre ledit échantillon en contact avec un radical indicateur capable de lier spécifiquement le polypeptide de SEQ ID NO : 43 dans laquelle X₁ représente V ou A et X₂ représente A ou T et X₃ représente T ou A, où le radical indicateur est en outre capable de lier spécifiquement des cellules intactes de *Campylobacter jejuni,* et
b. déterminer si un signal a été généré par le radical indicateur, détectant de cette manière si ledit échantillon contient *Campylobacter jejuni.*

36. Procédé selon la revendication 35, dans lequel ledit radical indicateur ne passe pas à travers la membrane externe d'une cellule de *Campylobacter jejuni.*

37. Procédé selon l'une quelconque des revendications 35 ou 36, dans lequel ledit radical indicateur est constitué de ou comprend un anticorps, tel qu'un anticorps tel que défini dans l'une quelconque des revendications 12 à 17.

38. Procédé d'identification d'un inhibiteur du polypeptide de SEQ ID NO : 43 dans laquelle X₁ représente V ou A et X₂ représente A ou T et X₃ représente T ou A, comprenant les étapes consistant à
a. fournir deux cellules qui diffèrent dans le taux du polypeptide de SEQ ID NO : 43 dans laquelle X₁ représente V ou A et X₂ représente A ou T et X₃ représente T ou A,
b. déterminer la sensibilité desdites cellules à un inhibiteur putatif, par exemple par un test de croissance, dans lequel l'inhibiteur putatif n'est pas capable de passer à travers la membrane externe d'une cellule de *Campylobacter jejuni,* et
c. déterminer si lesdites deux cellules sont affectées différemment par la présence dudit inhibiteur putatif.

39. Procédé d'identification d'un partenaire de liaison dérivé de l'hôte du polypeptide de SEQ ID NO : 43 dans laquelle X₁ représente V ou A et X₂ représente A ou T et X₃ représente T ou A ou d'un fragment antigénique choisi dans le groupe comprenant SEQ ID NO : 74 à 81, comprenant les étapes consistant à
a. fournir des membranes de l'hôte purifiées et séparées par électrophorèse transférées sur une membrane,
b. incuber lesdites membranes des l'hôte avec le polypeptide de SEQ ID NO : 43 dans laquelle X₁ représente V ou A et X₂ représente A ou T et X₃ représente T ou A ou un fragment antigénique choisi dans le groupe comprenant SEQ ID NO : 74 à 81,
c. détecter la liaison en utilisant des anticorps spécifiques du polypeptide de SEQ ID NO : 43 dans laquelle X₁ représente V ou A et X₂ représente A ou T et X₃ représente T ou A ou d'un fragment antigénique choisi dans le groupe comprenant SEQ ID NO : 74 à 81, et
d. identifier le partenaire de liaison par élution à partir de ladite membrane de transfert et analyse subséquente.
